# EUROPEAN PATENT APPLICATION

(11) **EP 2 460 891 A1**
(43) Date of publication of application: **06.06.2012**
(21) Application number: 10194061.7
(22) Date of filing: 07.12.2010
(51) Int. Cl.: C12Q 1/68

(54) **Prognostic marker for mamma carcinoma**

(30) Priority: 06.12.2010 EP 10193883
(71) Applicant: Medizinische Hochschule Hannover, 30625 Hannover (DE)
(72) Inventor: Dörk-Bousset, Thilo, 30629 Hannover (DE); Heuser, Michael, 30655 Hannover (DE); Damm, Frederik Martin Georges, 30451 Hannover (DE); Ganser, Arnold, 30559 Hannover (DE); Park-Simon, Tjoung-Won, 30559 Hannover (DE); Hillemanns, Peter, 30625 Hannover (DE)
(74) Representative: Taruttis, Stefan Georg

(57) **Abstract**

The invention provides an analytical method for the determination of the susceptibility of a human patient suffering from breast cancer to anti-cancer treatment by providing an analytical method for determining the genetic predisposition of a patient in respect of susceptibility, e.g. responsiveness, to anti-cancer treatment, especially treatment using at least one chemotherapeutical agent and/or at least one anti-hormonal agent on the basis of analysis of a genetic marker.

## Description

The invention relates to the analysis of a genetic marker for use as a prognostic marker in breast cancer, and to the analysis, and to the genetic marker, respectively, as an analyte for use in the selection of a therapy, as well as to an analytical method identifying the marker in breast cancer patients, especially for use in the prediction of the suitability of a treatment for a selected sub-group of patients. Further, the invention relates to the use of therapeutic agents for the long-term treatment, e.g. in a period of at least 3 to 5 years following initial cancer treatment or cancer diagnosis, especially in a period of at least 4 to 10 years following initial cancer treatment or cancer diagnosis, to improve the long-term treatment of breast cancer in a selected sub-group of patients, who are at least heterozygous for the genetic marker. The invention is based on the finding that in lobular breast cancer patients, one allele of the genetic marker is correlated with a poorer treatment prognosis, and hence the sub-group of patients requiring the administration of pharmaceutically active compounds as a medicament for treatment and/or prevention of lobular breast cancer, especially as a medicament for the prevention of recurring lobular breast cancer is characterized by the at least heterozygous presence of one allele of the marker. Interestingly, both alleles of the marker used in the invention give the same translation product, making the alleles a silent mutation.

### State of the art

Hollink et al., Blood 5951-5960 (2009) found that there is no relation of the status of the marker Ref SNP ID rs16754 to the course of cancer in patients, because the alleles of the marker Ref SNP ID rs16754 have no influence on the amino acid sequence of the protein produced from the WT1 gene concerned.

Li and Man, Cancer Biomark. 109-116 (2009) describe that the expression of the WT1 gene is changed in breast cancer cells. Miyoshi et al., Clin. Cancer Res. 1167-1171 (2002) relate the expression level of the WT1 gene to a poor prognosis in breast cancer patients.

Arpino et al., Breast Cancer Res. R149-156 (2004) found that invasive lobular breast cancer was more likely to occur in older patients, larger in size, positive for estrogen receptor and for progesterone receptor, to have a lower S-phase fraction, to be negative for HER-2, p53, and EGF-receptor.

### Objects of the invention

In view of the importance of adapting the therapeutic treatment to the type of breast cancer, it is the object of the invention to provide for a diagnostic marker that is predictive of the suitability of a therapeutic treatment. Preferably, the diagnostic marker is independent from known diagnostic findings.

It is a further object to provide an analytical method for use for the identification of breast cancer patients who can be treated successfully by standard therapy and for the identification of breast cancer patients who have a higher risk of relapse, e.g. 4 to 10 years after an initial cancer treatment, and who are therefore in need of additional treatment during this period. Accordingly, it is an object to provide a pharmaceutical composition for use as a medicament for treatment against the cancer in the patients that have a high risk of relapse, e.g. as a medicament for treatment within 4 to 10 years after an initial cancer treatment.

### General description of the invention

The invention attains the above-mentioned objects by the features of the claims, and especially by providing an analytical method for the determination of the susceptibility of a human patient suffering from breast cancer to anti-cancer treatment, e.g. by providing an analytical method for determining the genetic predisposition of a patient in respect of susceptibility, e.g. responsiveness, to anti-cancer treatment, especially treatment using at least one chemotherapeutical agent and/or at least one anti-hormonal agent. In greater detail, the invention provides the use of the marker Ref SNP ID rs16754 of the human WT1 gene, which is located on chromosome 11p15 (Wilms' tumour 1 gene) as an indicator in the diagnosis of nucleic acid samples originating from breast cancer patients, especially of lobular breast cancer patients, more especially in hormone receptor positive patients, as it has been found that the presence of at least one minor allele of this marker correlates with an increased risk of relapse in these patients. Further, the invention provides for the use of additional genetic markers in these embodiments, as these additional markers have been found to be coupled to the marker Ref SNP ID rs16754 of the human WT1 gene. Accordingly, for the purposes of the invention, the reference to the marker Ref SNP ID rs16754 includes the reference to the group comprising or consisting of coupled markers, which are given in Table 1.

**Table 1: Markers coupled to the marker Ref SNP ID rs16754**

| marker | chromosome position | D' | r² | LOD | minor allele | major allele |
|---|---|---|---|---|---|---|
| rs5030277 | 32374245 | 1 | 0.88 | 17.86 | A | T |
| rs5030280 | 32374008 | 1 | 0.876 | 17.33 | C | T |
| rs5030281 | 32373451 | 1 | 0.881 | 18.17 | T | C |
| rs5030310 | 32367816 | 1 | 0.882 | 18.32 | T | A |
| rs11031755 | 32351574 | 1 | 0.882 | 18.32 | T | c |
| rs11031747 | 32343797 | 0.933 | 0.816 | 15.67 | | |
| rs3858439 | 32343490 | 0.936 | 0.824 | 16.62 | C | A |
| rs3858438 | 32343130 | 0.936 | 0.824 | 16.62 | | |
| rs11031746 | 32342995 | 0.936 | 0.824 | 16.62 | C | A |
| rs11031745 | 32342895 | 0.936 | 0.824 | 16.62 | T | C |
| rs11031744 | 32342815 | 0.936 | 0.824 | 16.62 | T | C |
| rs3858436 | 32342153 | 1 | 0.875 | 16.89 | G | A |
| rs2418901 | 32341389 | 1 | 0.823 | 16.44 | G | A |

In Table 1, the chromosome position of coupled markers relates to the nucleotide position on human chromosome 11 (genome build 36.3), on which the chromosome position of marker rs16754 at nt 32374521 is a reference, in relation to which the positions of markers coupled to rs16754 are located on the WT1 gene. D' is the linkage disequilibrium (D), r is the correlation coefficient, and LOD is the LOD score estimating the recombination frequency. The alleles of the markers of table 1 that are coupled to the minor allele of marker rs16754, which due to the coupling are also termed minor alleles. Accordingly, for the purposes of the invention, reference to the minor allele of the marker Ref SNP ID rs16754 includes the group comprising or consisting of minor alleles of the coupled markers of table 1. Accordingly, the group comprising, preferably consisting of rs16754 and the coupled markers of table 1, especially their minor alleles form one group of markers which are indicative of lobular breast cancer patients, especially node-positive patients, who are in need of a more intense, especially a more extended treatment period, e.g. during or following 4 to 10 years after initial treatment or diagnosis, which group of markers is located at positions 32374245 to 32341389 on human chromosome 11 (genome build 36.3), wherein the members of the group of markers are closely coupled to one another, especially to marker rs16754 at position 32374521.

From Table 1, the following markers are preferred for the embodiments of the invention in addition and/or in the alternative to Ref SNP ID rs16754: rs16754, rs5030277, rs5030280, rs5030281, rs5030310, and/or rs11031755, the minor alleles of which are very closely coupled the minor allele of Ref SNP ID rs16754.

It has been found that the at least heterozygous allele G of the marker Ref SNP ID rs16754 is an indicator of a poor diagnosis in breast cancer patients, especially in lobular breast cancer patients, and therefore is an indicator identifying patients in need of intense cancer therapy and/or in need of long-term therapy, e.g. in the period of e.g. 4 to 15 years, especially 5 to 10 years after an initial cancer treatment, leading to a reduction in the proportion of relapse or progression rate of cancer. Accordingly, the analytical method of the invention allows to identify patients with the homozygous major allele of the marker Ref SNP ID rs16754 for less severe cancer treatment, because this group has a generally better prognosis, especially for long-term survival, whereas the analytical method allows to identify those patients who are in need of a more intense and/or more extended treatment, especially 4 to 10 years after initial cancer treatment, who are the patients having at least one minor allele of the marker Ref SNP ID rs16754.

In the process for analysis, the DNA from a breast cancer patient, preferably a node-positive lobular breast cancer patient, is analysed to determine the alleles of the marker Ref SNP ID rs16754 and/or of at least one of the markers coupled to Ref SNP ID rs16754, which coupled markers are given herein. As a result of the process, the absence or presence of at least one minor allele of at least one of the markers is determined.

It is a specific advantage of the analytical method of the invention to allow the identification of these groups of patients on the basis of a parameter, namely on the basis of the presence of the minor allele of the marker Ref SNP ID rs16754, which is independent from other analytical parameters, e.g. hormone receptor status (especially independent form the status of one of ER , PR and HER2), nodal status, tumour grade, and age in multivariate analysis.

Accordingly, the invention provides an analytical method determining the alleles of the marker Ref SNP ID rs16754 in a lobular breast cancer patient, e.g. followed by the step of selecting a method of treatment in dependence on the status of alleles of the marker Ref SNP ID rs16754, which status preferably is the at least heterozygous presence of the G-allele of the marker Ref SNP ID rs16754. Further, the invention provides pharmaceutical compositions for use as a medicament, i.e. for use as an active therapeutic agent in a pharmaceutical composition, especially a hormone receptor blocking agent, in the treatment of lobular breast cancer patients, which patients are characterized by the at least heterozygous allele G of the marker Ref SNP ID rs16754, e.g. as determined from a nucleic acid sample, e.g. total DNA obtained from a cell-containing sample of the patient.

Preferably, the presence of alleles of the marker Ref SNP ID rs16754 is determined by an analytical method comprising the step of amplification of a DNA section from a DNA sample obtained from the patient, preferably a total genomic DNA sample of the patient, e.g. by PCR, optionally with the step of analysing the nucleic acid sequence of the amplificate for identifying the nucleotide present in marker Ref SNP ID rs16754, e.g. the presence of the allele G, also termed minor allele or the presence of the allele A, which is also termed major allele, e.g. by DNA sequencing. Accordingly, the invention also relates to the use of a DNA amplificate generated in vitro by amplification of a DNA section of a patient's nucleic acid, e.g. a total DNA preparation or a total nucleic acid preparation isolated from a sample of the patient, for use as an indicator for identifying a sub-group of lobular breast cancer patients, who e.g. would benefit from or require intense cancer therapy and/or long-term therapy, e.g. in the period of e.g. 4 to 15 years, especially more than 5 or 6 years to 10 years after an initial cancer treatment. Preferably, the amplificate of the patient DNA containing the marker Ref SNP ID rs16754 for use as a diagnostic indicator, e.g. as a diagnostic indicator substance identifying a lobular breast cancer patient, especially as a patient requiring intense cancer therapy and/or in need of long-term therapy, e.g. in the period of e.g. 4 to 15 years, especially 5 to 10 years after an initial cancer treatment, is an amplificate having e.g. a length of 10 to 1500 bp, and is generated by PCR using at least one pair of primers which is specific for the DNA section. Specific primer pairs for amplification of a DNA section from total patient DNA, e.g. total genomic DNA, can be designed by standard methods, and processes for identifying the allele of marker Ref SNP ID rs16754 as G and/or A are also generally known, e.g. DNA sequencing or specific hybridisation of the amplificate containing the marker, which amplificate was obtained from the genomic DNA as a template for PCR.

The marker including the reference number Ref SNP ID rs16754 is e.g. available at NCBI databank (build 129), and is also termed C1107 A>G at GenBank entry NM/024426.3 (NM-024426.3: c.1107 A>G). The nucleotide sequence of the wt1 gene according to NM024426 is enclosed as SEQ ID NO 1, wherein nucleotides 197 to 1750 contain coding sequence, with exon 7 at nucleotides 1295 to 1445, and marker Ref SNP ID rs16754 at position 1303, which corresponds to nucleotide No 1107 of the coding sequence section (nt 197 to 1750). The amino acid sequence encoded by exon 7 is given as SEQ ID NO 2, irrespective of the natural reading frame.

Preferably, the analytical method of the invention further includes at least one additional analytical step for analysis of the lobular breast cancer, which analytical step is selected from the group comprising or consisting of an analysis
in respect of its estrogen receptor status, e.g. negative or positive, as can be determined by standard analytical processes, e.g. by immuno histochemistry,
in respect of its progesteron receptor status, e.g. negative or positive as can be determined by standard analytical processes, e.g. by immuno histochemistry,
in respect of its lymph node (node) status, e.g. node negative or node positive as can be determined by standard analytical processes, e.g. by histopathology,
in respect of its HER-2 status, e.g. negative or positive as can be determined by standard analytical processes, e.g. by immuno histochemistry,
in respect of its tumour grade status, e.g. grade 2 vs. grade 1 and grade 3 vs. grade 1, e.g. can be determined by pathologic evaluation,
and in respect of patient age, e.g. younger than 50 years vs. 50 years and older.

It has been found that especially patients with lobular breast cancer who at least heterozygously have the minor allele of the marker Ref SNP ID rs16754 who addditionally have a positive hormone receptor status, e.g. selected from estrogen receptor status, progesteron receptor status and/or HER-2 status have a significantly worse prognosis, and are therefore in need for additional therapy, e.g. in the period of 4 to 10 years following initial cancer treatment. Accordingly, these patients are the preferred patients of the invention, e.g. lobular breast cancer patients carrying at least one minor allele of the marker Ref SNP ID rs16754 and having a tumour that is positive for hormone receptor status, e.g. selected from estrogen receptor status, progesteron receptor status and/or HER-2 status, especially estrogen receptor status and progesteron receptor status.

### Detailed description of the invention

The invention is now described in greater detail with reference to the figures showing graphical representations of analytical results between breast cancer patients who are homozygous for the major allele (A/A) or have at least one minor allele (A/G and G/G) of the marker Ref SNP ID rs16754, namely in
- Fig. 1 the OS in univariate analysis of all breast cancer patients (n=8094) analyzed,
- Fig. 2 the OS for lobular breast cancer patients only,
- Fig. 3 the breast cancer specific survival in lobular breast cancer patients,
- Fig. 4 the OS for ER positive and/or PR positive patients,
- Fig. 5 the OS for ER negative and/or PR negative patients,
- Fig. 6 the OS for lobular breast cancer patients younger than 50 years, and in
- Fig. 7 the OS for lobular breast cancer patients older than 50 years.
according to the status of Ref SNP ID rs16754.

In the figures, year 0 of the time scale denotes the initial diagnosis and/or onset of cancer treatment.

The present invention is based on a large-scale medical study involving 8337 female breast cancer patients, in whom the alleles of the marker Ref SNP ID rs16754 were analysed in PBMC (peripheral mononuclear blood cells) and relevance of the alleles was assessed in a univariate and multivariate analyses considering age, tumour grade, lymph node involvement (nodal status), estrogen receptor status, progesterone receptor status, and HER-2 status. Generally, the data that were analysed originate from patients undergoing similar treatments, e.g. treatment was similar, irrespective of lobular or ductal breast cancer, and irrespective of the alleles of the marker Ref SNP ID rs16754.

It was found that the minor allele of the marker Ref SNP ID rs16754 is heterozygous in 25.2% of the patients and homozygous in 2.8% of the patients. Patient characteristics and pathological findings were similarly distributed between patients who are homozygous for the major allele (A/A) of the marker Ref SNP ID rs16754, and those patients who are at least heterozygous for the minor allele of the marker Ref SNP ID rs16754, i.e. heterozygous (A/G) or homozygous (G/G) for the minor allele (G).

Only in patients (n=1101) having lobular breast cancer, it was determined that the alleles of the marker Ref SNP ID rs16754 were independently predictive for overall survival (OS) in multivariate analysis (HR 1.44, 95% CI 1.06-1.94, P = 0.018), and for breast cancer specific survival with HR 1.49, 95% CI 1.07-2.07, P= 0.018, wherein HR is the hazard ratio, CI is the confidence interval, and P is the P-value indicating significance, whereas in other histological subtypes, e.g. in ductal breast cancer, a predictive value of the marker Ref SNP ID rs16754 was not found . The linkage of a minor allele of the marker Ref SNP ID rs16754 with a poor prognosis, and the need for more intense and/or longer administration of anti-cancer medicaments became more pronounced at 4 or 5 years after initial diagnosis and/or after initial treatment, and was most pronounced in hormone receptor positive and in younger lobular breast cancer patients.

Preferably, compounds for use as a medicament in the treatment of lobular breast cancer patients having at least one minor allele of the marker Ref SNP ID rs16754 are selected from compounds used as medicaments in the treatment of hormone receptor positive breast cancer patients, especially tamoxifen, ovarian suppression agents, aromatase inhibitors, and irreversible estrogen receptor inhibitors or combinations of these, especially Tamoxifen, Anastrozol, Letrozol, and Exemestan.

### Example 1: Analysis of the marker Ref SNP ID rs16754

Analyses of the alleles of the marker Ref SNP ID rs16754 were performed using the commercially available (Applied Biosystems, Assay ID C_12034416_10) PCR-based assay specific for marker Ref SNP ID rs16754. Generally, total DNA was isolated from PBMC of breast cancer patients and subjected to PCR in microtiter plates at an annealing temperature of 60°C using the TaqMan protocol supplied by the manufacturer. For amplification, a forward primer of SEQ ID NO 3 and a backward primer of SEQ ID NO 4 were used. Detection and assessment was made with the Sequence Detection Software supplied by the manufacturer.

For statistical analyses, median follow-up time for survival was calculated according to Korn, Stat Med 5: 255-260 (1986). Overall survival (OS) endpoints were death (failure) and alive at last follow-up (generally censored at 10 years), measured from the date of entry into the study. Pair-wise comparisons of variables for exploratory purposes were done using the Kolmogorov-Smirnov test for continuous variables and the Chi-squared test for categorical variables. Estimation of distribution of OS and comparison of differences between survival curves was by the Kaplan-Meier method and the log-rank test, respectively. A Cox proportional hazards model for OS was developed for multivariate analysis and stratified by clinical study. The proportional hazards assumption was checked by scaled Schoenfeld residuals. Variables with P of 0.1 or below in univariate analysis were included into the model. Status of the marker Ref SNP ID rs16754 (homozygous for major allele vs. at least one minor allele), nodal status (node positive vs. node negative), estrogen receptor (ER) status (ER positive vs. ER negative), progesterone receptor (PR) status (PR positive vs. PR negative), HER-2 status (HER-2 positive vs. HER-2 negative), tumour grade (grade 2 vs. grade 1, and grade 3 vs. grade 1), and age (below 50 years vs. at least 50 years) were the variables considered.

Incomplete (more than 60%) data sets of patients were used for the multivariate Cox model following imputation of missing data using the aregImpute function of the R software package Hmisc, version 3.7, allowing the algorithm to use data from all other variables, except for the age. This procedure models the distribution of each variable present to the other variables with a predictive mean matching, and using bootstrapping to generate multiple imputations by approximating samples from the posterior predictive distributions of missing values to account for all aspects of uncertainty. Following an initial 10 data sets, a further 50 data sets were imputed. Using a logistic regression model, associations between variables and response to induction therapy were analysed. For relevance of quantitative results, odds ratios (OR) and hazard ratios (HR) were calculated at 95% CI, and the two-sided level of significance was set at *P* less than 0.05. For statistical analyses, the SPSS programme version 17.0 and R programme version 2.10 were used.

It was found that 71.9% of patients were homozygous for the major allele of the marker Ref SNP ID rs16754 (A/A), 25.2% were heterozygous (A/G), and 2.8% were homozygous for the minor allele of the marker Ref SNP ID rs16754 (G/G). Generally, this allele distribution corresponds to that of the general European population. When comparing pre-treatment clinical and molecular characteristics of the patients, including age, bilateral disease, tumour stage and grade, nodal status, tumour histology, ER status, PR status, ER/PR status, HER-2 status or ER/PR/HER-2 status, no significant differences were found between patients who are homozygous for the major allele to patients having at least one minor allele of the marker Ref SNP ID rs16754.

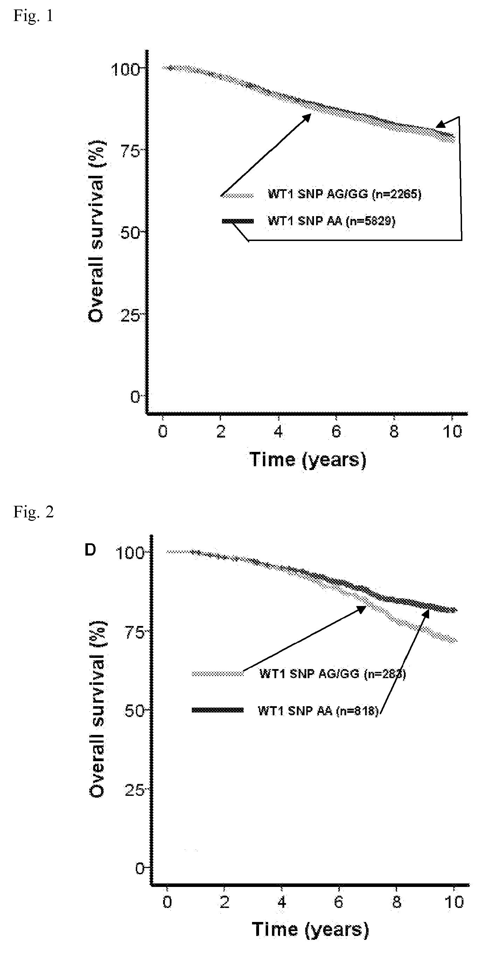

When analysing overall survival (average 9.67 years, range 0.3 to 10 years) of all breast cancer patients (n=8094; P=0.54), no significant difference was found between homozygous major alleles and at least heterozygous minor alleles of the marker Ref SNP ID rs16754, as shown in Fig. 1. In ductal carcinoma patients, OS was not significantly different between patients with at least one minor allele and patients homozygous for the major allele (lymph node negative P= 0.54, lymph node positive P=0.46, ductal carcinoma P=0.65)

Further, OS was not significantly different in node positive (n=2825) patients between patients homozygous for the major allele and patients having at least one minor allele of the marker Ref SNP ID rs16754 (P=0.41).

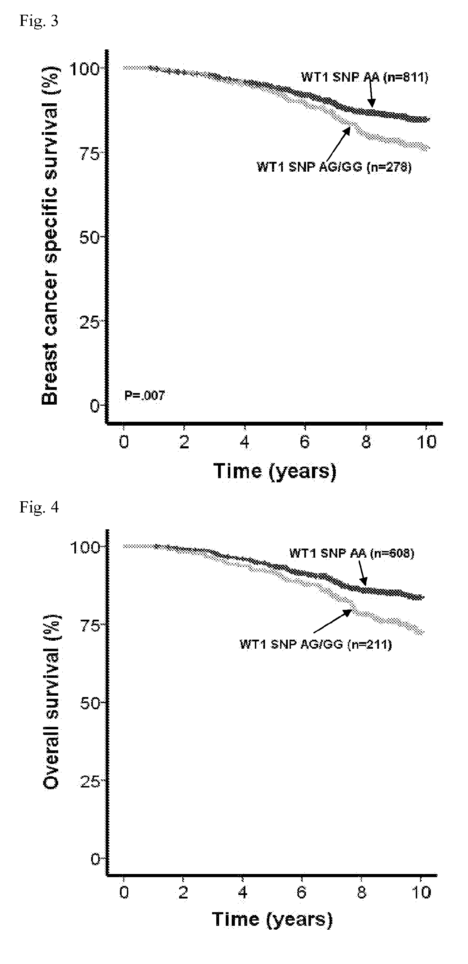

However, it was found that OS of lobular breast cancer patients having at least one minor allele of the marker Ref SNP ID rs16754 was significantly shorter (HR=1.54, 95% CI 1.15 - 2.06, P=0.004) compared to lobular breast cancer patients having the major allele of the marker Ref SNP ID rs16754 homozygously, as shown in Fig. 2. Lobular breast cancer patients having at least one minor allele of the marker Ref SNP ID rs16754 showed a 5-year OS of 88% and a 10-year OS of 71% compared to a 5-year OS of 90% and a 10-year OS of 81 % for lobular breast cancer patients having the major allele of the marker Ref SNP ID rs16754 homozygously. It was found that the baseline characteristics were similarly distributed between patients homozygous for the major allele and patients having the minor allele of the marker Ref SNP ID rs16754, respectively, except for a higher occurrence of node positive breast cancer in patients with at least one minor allele (41% vs. 35%, P=0.058). Importantly, BCSS was significantly shorter in lobular breast cancer patients with at least one minor allele of SNP rs16754 compared to major allele homozygous patients (HR 1.55, 95% CI 1.13 - 2.14, P= 0.007) as shown in Fig. 3.

In multivariate analysis of variables having P=0.1 or below in univariate analysis, it was found that presence of at least one minor allele of the marker Ref SNP ID rs16754 in lobular breast cancer patients is associated and predictive of an increased hazard of death (HR 1.46, 95% CI 1.08 - 1.96, P=0.013). Details are shown in the following Table 2, wherein HR above 1 indicates an increased risk for the respective variable, and HR below 1 indicates a decreased risk.

**Table 2: Multivariate analysis for OS in lobular breast cancer patients (n=1089)**

| Variable | HR | 95% CI | P |
|---|---|---|---|
| at least one minor allele of the marker Ref SNP ID | 1.49 | 1.07-2.07 | 0.018 |
| rs16754 | | | |
| vs. | | | |
| homozygousity for major allele of the marker Ref SNP ID | | | |
| rs16754 | | | |
| node positive vs. node negative | 3.24 | 2.27-4.62 | <0.001 |
| ER positive vs. ER negative | 0.82 | 0.48-1.41 | 0.47 |
| PR positive vs. PR negative | 0.58 | 0.39-0.88 | 0.01 |
| HER-2 positive vs. HER-2 negative | 1.24 | 0.54-2.85 | 0.62 |
| grade 2 vs. grade 1 | 1.42 | 0.9-2.23 | 0.13, |
| grade 3 vs. grade 1 | 2.52 | 1.31-4.83 | 0.006 |
| age ≥ 50 years vs. age < 50 years | 0.73 | 0.51-1.05 | 0.09 |

Herein, a hazard ratio (HR) above 1 indicates an increased risk for the first category listed.

### Example 2: Medicament for treatment of lobular breast cancer in patients having a minor allele of the marker Ref SNP ID rs16754

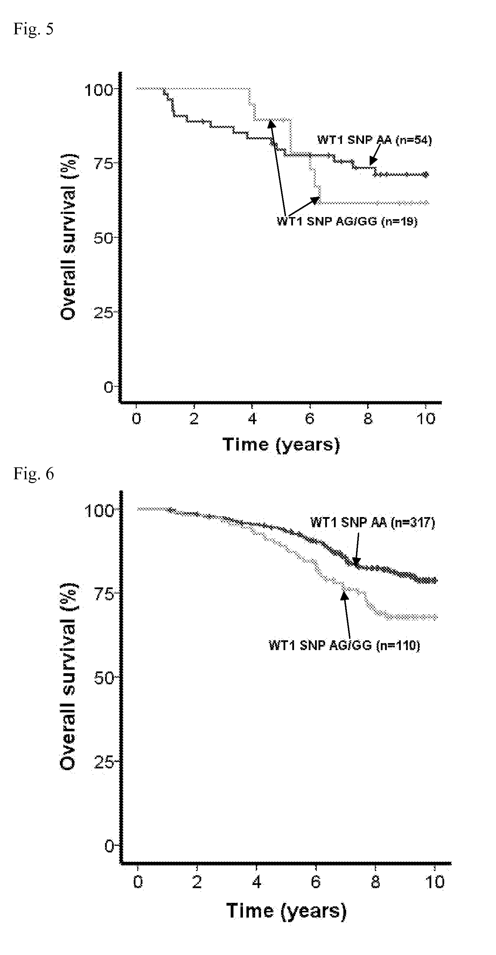

In the data analysis it became clear that the presence of at least one minor allele of the marker Ref SNP ID rs16754 in lobular breast cancer patients especially in a period of approx. 4 - 5 years after initial cancer diagnosis and treatment is correlated with a negative prognosis, i.e. a lower OS. When analysing the correlation of the alleles of the marker Ref SNP ID rs16754 to hormone receptor positive and negative patients, it was found that the presence of the minor allele was correlated with a reduced OS in hormone receptor positive patients (P=0.004, Fig. 4), but not in hormone receptor negative patients (P=0.75, Fig. 5).

In contrast to the hormone negative patients, the hormone receptor positive patients were generally treated with a hormone ablation therapy, which typically involves the administration of Tamoxifen. From the correlation of the presence of the minor allele of the marker Ref SNP ID rs16754 in lobular breast cancer patients with a reduced OS in those patients who due to the negative hormone receptor status generally did not receive treatment in the period 5 years after initial diagnosis, and the observation that presence of the minor allele is not correlated with a reduced OS in patients that receive treatment hormone ablation treatment, it can be concluded that lobular breast cancer patients having at least one minor allele of the marker Ref SNP ID rs16754 benefit from treatment with anti-hormone receptor pharmaceutical compounds irrespective of the presence of infiltrated lymph nodes.

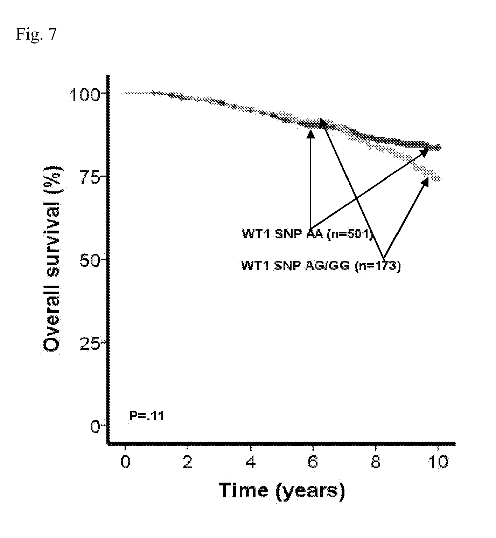

The above finding of a positive therapeutic effect of hormone ablation therapeutics in lobular breast cancer patients having at least one minor allele of the marker Ref SNP ID rs16754 was confirmed in menopausal patients. Statistical analysis showed the correlation of the presence of at least one minor allele of the marker Ref SNP ID rs16754 with reduced OS in younger patients (age of less than 50 years, mean age 43.7 years), who generally do not receive hormone ablation therapy, compared to patients who are homozygous for the major allele of the marker Ref SNP ID rs16754 (P=0.01, Fig. 6). The difference in OS was less evident in older patients (age above 50 years, mean age 59.1 years), who generally receive hormone ablation therapy, between lobular breast cancer patients having at least one minor allele of the marker Ref SNP ID rs16754 and patients who are homozygous for the major allele of the marker Ref SNP ID rs16754 (P=0.13, Fig. 7).

Hence, the invention also provides for compounds, e.g. selected from the group of pharmaceutical compounds used in hormone ablation therapy, e.g. a hormone receptor blocking agent, especially Tamoxifen, Anastrozol, Letrozol, Exemestan, for use as a medicament in the treatment of lobular breast cancer patients having at least one minor allele of the marker Ref SNP ID rs16754, especially for administration in a period of time after 4 to 5 years, e.g. up to 10 years, following initial breast cancer diagnosis or following initial breast cancer treatment.

Further, the analysis of OS in node positive lobular breast cancer patients revealed no significant difference between patients being homozygous for the major allele of the marker Ref SNP ID rs16754 and patients having at least one minor allele of the marker Ref SNP ID rs16754. When considering that generally the presence of the minor allele of the marker Ref SNP ID rs16754 is correlated with a reduced OS, the finding that node positive breast cancer patients generally receive cancer treatment by irradiation and/or chemotherapy to a higher extent than node negative patients demonstrates that especially node positive lobular breast cancer patients having at least one minor allele of the marker Ref SNP ID rs16754 benefit from the additional or harsher cancer treatment, e.g. irradiation and/or chemotherapy.

Hence, the invention also provides for compounds, e.g. selected from the group of pharmaceutical compounds used in cancer therapy, e.g. Tamoxifen, Anastrozol, Letrozol or Exemestan for use as a medicament in the treatment of lobular breast cancer patients having at least one minor allele of the marker Ref SNP ID rs16754 or having the coupled allele of a marker coupled to the marker Ref SNP ID rs16754, especially for administration in a period of time after 4 to 5 years following initial breast cancer diagnosis or treatment, especially those patients who are node positive and/or patients who are node negative.

## Claims

1. Process for the analysis of the susceptibility of a human patient to anti-cancer treatment, **characterized by** analysing the alleles of the marker Ref SNP ID rs16754 and/or of the alleles of at least one marker of the group of markers comprising rs5030277, rs5030280, rs5030281, rs5030310, rs11031755, rs11031747, rs3858439, rs3858438, rs11031746, rs11031745, rs11031744, rs3858436, rs2418901, in a nucleic acid sample obtained from a human suffering from lobular breast cancer.

2. Process according to claim 1, **characterized in that** the breast cancer has at least one positive hormone receptor status.

3. Process according to claim 2, **characterized in that** the positive hormone receptor status is at least one of estrogen receptor status, progesteron receptor status and/or HER-2 status.

4. Process according to one of the preceding claims, **characterized in that** the process is for use in the selection of a therapy against lobular breast cancer.

5. Process according to one of the preceding claims, **characterized in that** the nucleic acid sample is provided, and the alleles of the marker Ref SNP ID rs16754 and/or the alleles of at least one of the markers of the group comprising rs5030277, rs5030280, rs5030281, rs5030310, rs11031755, rs11031747, rs3858439, rs3858438, rs11031746, rs11031745, rs11031744, rs3858436, rs2418901 are determined, and the hormone receptor status of the patient is determined.

6. Process according to one of the preceding claims, **characterized in that** the hormone receptor status of the cancer is determined.

7. Use of a kit of parts containing oligonucleotides which are complementary to at least a section of the human WT1 gene corresponding to SEQ ID NO 1 as reagents for the analysis of the allele of the marker Ref SNP ID rs16754 and/or of the allele of at least one of the markers of the group comprising rs5030277, rs5030280, rs5030281, rs5030310, rs11031755, rs11031747, rs3858439, rs3858438, rs11031746, rs11031745, rs11031744, rs3858436, rs2418901 in a process according to one of the preceding claims in a nucleic acid sample obtained from a human patient suffering from lobular breast cancer.

8. Pharmaceutical composition containing Tamoxifen, Anastrozol, Letrozol or Exemestan as an active compound for use as a medicament in the treatment of lobular breast cancer in patients who are **characterized by** bearing the allele G in marker Ref SNP ID rs16754 at least heterozygously, or the minor allele of at least one of the markers of the group comprising rs5030277, rs5030280, rs5030281, rs5030310, rs11031755, rs11031747, rs3858439, rs3858438, rs11031746, rs11031745, rs11031744, rs3858436, rs2418901 at least heterozygously.

9. Pharmaceutical composition according to claim 8, **characterized in that** the treatment is for at least 6 years to 10 years following the detection of breast cancer.

10. Pharmaceutical composition according to claim 8 or 9, **characterized in that** the patients have received treatment against breast cancer 5 to 10 years previously.

11. Pharmaceutical composition according to one of claims 8 to 10, **characterized by** containing a hormone receptor blocking agent as the pharmaceutically active compound.

12. Use of a process for analysis of the alleles of the marker Ref SNP ID rs16754 or of the alleles of at least one of the markers of the group comprising rs5030277, rs5030280, rs5030281, rs5030310, rs11031755, rs11031747, rs3858439, rs3858438, rs11031746, rs11031745, rs11031744, rs3858436, rs2418901 in a nucleic acid sample, **characterized in that** the nucleic acid sample was obtained from a human patient suffering from lobular breast cancer, in the selection of a therapy in a period of 3 to 10 years following the detection of breast cancer.

13. Use of an amplified section of the human WT1 gene, which section is obtainable by enzymatic DNA amplification from a DNA sample originating from a lobular breast cancer patient, which section comprises the marker Ref SNP ID rs16754 and/or at least one of the markers from the group comprising rs5030277, rs5030280, rs5030281, rs5030310, rs11031755, rs11031747, rs3858439, rs3858438, rs11031746, rs11031745, rs11031744, rs3858436, rs2418901, as an indicator substance for identifying patients.

14. Use according to claim 13, wherein the patients are identified by the presence of at least one G-allele of the marker Ref SNP ID rs16754 and/or at least one minor allele of at least one of the markers of the group consisting of rs5030277, rs5030280, rs5030281, rs5030310, rs11031755, rs11031747, rs3858439, rs3858438, rs11031746, rs11031745, rs11031744, rs3858436, and rs2418901.

15. Use according to one of claims 13 to 14, **characterized in that** the amplified DNA section comprises nucleotides 32374245 to 32341389 of human chromosome 11.


SEQUENCE LISTING

<110>  Medizinische Hochschule Hannover

<120>  Prognostic marker for mamma carcinoma

<130>  M1031EP

<160>  4

<170>  PatentIn version 3.5

<210>  1
<211>  3029
<212>  DNA
<213>  Homo sapiens


<220>
<221>  CDS
<222>  (197)..(1750)
<223>  exon 7

<220>
<221>  misc_feature
<222>  (1303)..(1303)
<223>  SNP ID rs16754

<400>  1
ccaggcagct ggggtaagga gttcaaggca gcgcccacac ccgggggctc tccgcaaccc      60

gaccgcctgt ccgctccccc acttcccgcc ctccctccca cctactcatt cacccaccca     120

cccacccaga gccgggacgg cagcccaggc gcccgggccc cgccgtctcc tcgccgcgat     180

cctggacttc ctcttg ctg cag gac ccg gct tcc acg tgt gtc ccg gag ccg     232
                  Leu Gln Asp Pro Ala Ser Thr Cys Val Pro Glu Pro
                  1               5                   10

gcg tct cag cac acg ctc cgc tcc ggg cct ggg tgc cta cag cag cca       280
Ala Ser Gln His Thr Leu Arg Ser Gly Pro Gly Cys Leu Gln Gln Pro
        15                  20                  25

gag cag cag gga gtc cgg gac ccg ggc ggc atc tgg gcc aag tta ggc       328
Glu Gln Gln Gly Val Arg Asp Pro Gly Gly Ile Trp Ala Lys Leu Gly
    30                  35                  40

gcc gcc gag gcc agc gct gaa cgt ctc cag ggc cgg agg agc cgc ggg       376
Ala Ala Glu Ala Ser Ala Glu Arg Leu Gln Gly Arg Arg Ser Arg Gly
45                  50                  55                  60

gcg tcc ggg tct gag ccg cag caa atg ggc tcc gac gtg cgg gac ctg       424
Ala Ser Gly Ser Glu Pro Gln Gln Met Gly Ser Asp Val Arg Asp Leu
                65                  70                  75

aac gcg ctg ctg ccc gcc gtc ccc tcc ctg ggt ggc ggc ggc ggc tgt       472
Asn Ala Leu Leu Pro Ala Val Pro Ser Leu Gly Gly Gly Gly Gly Cys
            80                  85                  90

gcc ctg cct gtg agc ggc gcg gcg cag tgg gcg ccg gtg ctg gac ttt       520
Ala Leu Pro Val Ser Gly Ala Ala Gln Trp Ala Pro Val Leu Asp Phe
        95                  100                 105

gcg ccc ccg ggc gct tcg gct tac ggg tcg ttg ggc ggc ccc gcg ccg       568
Ala Pro Pro Gly Ala Ser Ala Tyr Gly Ser Leu Gly Gly Pro Ala Pro
    110                 115                 120

cca ccg gct ccg ccg cca ccc ccg ccg ccg ccg cct cac tcc ttc atc       616
Pro Pro Ala Pro Pro Pro Pro Pro Pro Pro Pro Pro His Ser Phe Ile

125                 130                 135                 140

aaa cag gag ccg agc tgg ggc ggc gcg gag ccg cac gag gag cag tgc       664
Lys Gln Glu Pro Ser Trp Gly Gly Ala Glu Pro His Glu Glu Gln Cys
                145                 150                 155

ctg agc gcc ttc act gtc cac ttt tcc ggc cag ttc act ggc aca gcc       712
Leu Ser Ala Phe Thr Val His Phe Ser Gly Gln Phe Thr Gly Thr Ala
            160                 165                 170

gga gcc tgt cgc tac ggg ccc ttc ggt cct cct ccg ccc agc cag gcg       760
Gly Ala Cys Arg Tyr Gly Pro Phe Gly Pro Pro Pro Pro Ser Gln Ala
        175                 180                 185

tca tcc ggc cag gcc agg atg ttt cct aac gcg ccc tac ctg ccc agc       808
Ser Ser Gly Gln Ala Arg Met Phe Pro Asn Ala Pro Tyr Leu Pro Ser
    190                 195                 200

tgc ctc gag agc cag ccc gct att cgc aat cag ggt tac agc acg gtc       856
Cys Leu Glu Ser Gln Pro Ala Ile Arg Asn Gln Gly Tyr Ser Thr Val
205                 210                 215                 220

acc ttc gac ggg acg ccc agc tac ggt cac acg ccc tcg cac cat gcg       904
Thr Phe Asp Gly Thr Pro Ser Tyr Gly His Thr Pro Ser His His Ala
                225                 230                 235

gcg cag ttc ccc aac cac tca ttc aag cat gag gat ccc atg ggc cag       952
Ala Gln Phe Pro Asn His Ser Phe Lys His Glu Asp Pro Met Gly Gln
            240                 245                 250

cag ggc tcg ctg ggt gag cag cag tac tcg gtg ccg ccc ccg gtc tat      1000
Gln Gly Ser Leu Gly Glu Gln Gln Tyr Ser Val Pro Pro Pro Val Tyr
        255                 260                 265

ggc tgc cac acc ccc acc gac agc tgc acc ggc agc cag gct ttg ctg      1048
Gly Cys His Thr Pro Thr Asp Ser Cys Thr Gly Ser Gln Ala Leu Leu
    270                 275                 280

ctg agg acg ccc tac agc agt gac aat tta tac caa atg aca tcc cag      1096
Leu Arg Thr Pro Tyr Ser Ser Asp Asn Leu Tyr Gln Met Thr Ser Gln
285                 290                 295                 300

ctt gaa tgc atg acc tgg aat cag atg aac tta gga gcc acc tta aag      1144
Leu Glu Cys Met Thr Trp Asn Gln Met Asn Leu Gly Ala Thr Leu Lys
                305                 310                 315

gga gtt gct gct ggg agc tcc agc tca gtg aaa tgg aca gaa ggg cag      1192
Gly Val Ala Ala Gly Ser Ser Ser Ser Val Lys Trp Thr Glu Gly Gln
            320                 325                 330

agc aac cac agc aca ggg tac gag agc gat aac cac aca acg ccc atc      1240
Ser Asn His Ser Thr Gly Tyr Glu Ser Asp Asn His Thr Thr Pro Ile
        335                 340                 345

ctc tgc gga gcc caa tac aga ata cac acg cac ggt gtc ttc aga ggc      1288
Leu Cys Gly Ala Gln Tyr Arg Ile His Thr His Gly Val Phe Arg Gly
    350                 355                 360

att cag gat gtg cga cgt gtg cct gga gta gcc ccg act ctt gta cgg      1336
Ile Gln Asp Val Arg Arg Val Pro Gly Val Ala Pro Thr Leu Val Arg
365                 370                 375                 380

tcg gca tct gag acc agt gag aaa cgc ccc ttc atg tgt gct tac cca      1384
Ser Ala Ser Glu Thr Ser Glu Lys Arg Pro Phe Met Cys Ala Tyr Pro
                385                 390                 395

ggc tgc aat aag aga tat ttt aag ctg tcc cac tta cag atg cac agc      1432
Gly Cys Asn Lys Arg Tyr Phe Lys Leu Ser His Leu Gln Met His Ser
            400                 405                 410

agg aag cac act ggt gag aaa cca tac cag tgt gac ttc aag gac tgt     1480
Arg Lys His Thr Gly Glu Lys Pro Tyr Gln Cys Asp Phe Lys Asp Cys
        415                 420                 425

gaa cga agg ttt tct cgt tca gac cag ctc aaa aga cac caa agg aga     1528
Glu Arg Arg Phe Ser Arg Ser Asp Gln Leu Lys Arg His Gln Arg Arg
    430                 435                 440

cat aca ggt gtg aaa cca ttc cag tgt aaa act tgt cag cga aag ttc     1576
His Thr Gly Val Lys Pro Phe Gln Cys Lys Thr Cys Gln Arg Lys Phe
445                 450                 455                 460

tcc cgg tcc gac cac ctg aag acc cac acc agg act cat aca ggt aaa     1624
Ser Arg Ser Asp His Leu Lys Thr His Thr Arg Thr His Thr Gly Lys
                465                 470                 475

aca agt gaa aag ccc ttc agc tgt cgg tgg cca agt tgt cag aaa aag     1672
Thr Ser Glu Lys Pro Phe Ser Cys Arg Trp Pro Ser Cys Gln Lys Lys
            480                 485                 490

ttt gcc cgg tca gat gaa tta gtc cgc cat cac aac atg cat cag aga     1720
Phe Ala Arg Ser Asp Glu Leu Val Arg His His Asn Met His Gln Arg
        495                 500                 505

aac atg acc aaa ctc cag ctg gcg ctt tga ggggtctccc tcggggaccg       1770
Asn Met Thr Lys Leu Gln Leu Ala Leu
    510                 515

ttcagtgtcc caggcagcac agtgtgtgaa ctgctttcaa gtctgactct ccactcctcc   1830

tcactaaaaa ggaaacttca gttgatcttc ttcatccaac ttccaagaca agataccggt   1890

gcttctggaa actaccaggt gtgcctggaa gagttggtct ctgccctgcc tacttttagt   1950

tgactcacag gccctggaga agcagctaac aatgtctggt tagttaaaag cccattgcca   2010

tttggtgtgg attttctact gtaagaagag ccatagctga tcatgtcccc ctgacccttc   2070

ccttcttttt ttatgctcgt tttcgctggg gatggaatta ttgtaccatt ttctatcatg   2130

gaatatttat aggccagggc atgtgtatgt gtctgctaat gtaaactttg tcatggtttc   2190

catttactaa cagcaacagc aagaaataaa tcagagagca aggcatcggg ggtgaatctt   2250

gtctaacatt cccgaggtca gccaggctgc taacctggaa agcaggatgt agttctgcca   2310

ggcaactttt aaagctcatg catttcaagc agctgaagaa aaaatcagaa ctaaccagta   2370

cctctgtata gaaatctaaa agaattttac cattcagtta attcaatgtg aacactggca   2430

cactgctctt aagaaactat gaagatctga gatttttttg tgtatgtttt tgactctttt   2490

gagtggtaat catatgtgtc tttatagatg tacatacctc cttgcacaaa tggaggggaa   2550

ttcattttca tcactgggag tgtccttagt gtataaaaac catgctggta tatggcttca   2610

agttgtaaaa atgaaagtga ctttaaaaga aaatagggga tggtccagga tctccactga   2670

taagactgtt tttaagtaac ttaaggacct ttgggtctac aagtatatgt gaaaaaaatg   2730

agacttactg ggtgaggaaa tccattgttt aaagatggtc gtgtgtgtgt gtgtgtgtgt   2790

gtgtgtgtgt gtgttgtgtt gtgttttgtt ttttaaggga gggaatttat tatttaccgt   2850

tgcttgaaat tactgtgtaa atatatgtct gataatgatt tgctctttga caactaaaat   2910

taggactgta taagtactag atgcatcact gggtgttgat cttacaagat attgatgata   2970

acacttaaaa ttgtaacctg catttttcac tttgctctca attaaagtct attcaaaag      3029

<210>  2
<211>  517
<212>  PRT
<213>  Homo sapiens

<400>  2

Leu Gln Asp Pro Ala Ser Thr Cys Val Pro Glu Pro Ala Ser Gln His
1               5                   10                  15

Thr Leu Arg Ser Gly Pro Gly Cys Leu Gln Gln Pro Glu Gln Gln Gly
            20                  25                  30

Val Arg Asp Pro Gly Gly Ile Trp Ala Lys Leu Gly Ala Ala Glu Ala
        35                  40                  45

Ser Ala Glu Arg Leu Gln Gly Arg Arg Ser Arg Gly Ala Ser Gly Ser
    50                  55                  60

Glu Pro Gln Gln Met Gly Ser Asp Val Arg Asp Leu Asn Ala Leu Leu
65                  70                  75                  80

Pro Ala Val Pro Ser Leu Gly Gly Gly Gly Gly Cys Ala Leu Pro Val
                85                  90                  95

Ser Gly Ala Ala Gln Trp Ala Pro Val Leu Asp Phe Ala Pro Pro Gly
            100                 105                 110

Ala Ser Ala Tyr Gly Ser Leu Gly Gly Pro Ala Pro Pro Pro Ala Pro
        115                 120                 125

Pro Pro Pro Pro Pro Pro Pro Pro His Ser Phe Ile Lys Gln Glu Pro
    130                 135                 140

Ser Trp Gly Gly Ala Glu Pro His Glu Glu Gln Cys Leu Ser Ala Phe
145                 150                 155                 160

Thr Val His Phe Ser Gly Gln Phe Thr Gly Thr Ala Gly Ala Cys Arg
                165                 170                 175

Tyr Gly Pro Phe Gly Pro Pro Pro Pro Ser Gln Ala Ser Ser Gly Gln
            180                 185                 190

Ala Arg Met Phe Pro Asn Ala Pro Tyr Leu Pro Ser Cys Leu Glu Ser
        195                 200                 205

Gln Pro Ala Ile Arg Asn Gln Gly Tyr Ser Thr Val Thr Phe Asp Gly
    210                 215                 220

Thr Pro Ser Tyr Gly His Thr Pro Ser His His Ala Ala Gln Phe Pro
225                 230                 235                 240

Asn His Ser Phe Lys His Glu Asp Pro Met Gly Gln Gln Gly Ser Leu
                245                 250                 255

Gly Glu Gln Gln Tyr Ser Val Pro Pro Pro Val Tyr Gly Cys His Thr
            260                 265                 270

Pro Thr Asp Ser Cys Thr Gly Ser Gln Ala Leu Leu Leu Arg Thr Pro
        275                 280                 285

Tyr Ser Ser Asp Asn Leu Tyr Gln Met Thr Ser Gln Leu Glu Cys Met
    290                 295                 300

Thr Trp Asn Gln Met Asn Leu Gly Ala Thr Leu Lys Gly Val Ala Ala
305                 310                 315                 320

Gly Ser Ser Ser Ser Val Lys Trp Thr Glu Gly Gln Ser Asn His Ser
                325                 330                 335

Thr Gly Tyr Glu Ser Asp Asn His Thr Thr Pro Ile Leu Cys Gly Ala
            340                 345                 350

Gln Tyr Arg Ile His Thr His Gly Val Phe Arg Gly Ile Gln Asp Val
        355                 360                 365

Arg Arg Val Pro Gly Val Ala Pro Thr Leu Val Arg Ser Ala Ser Glu
    370                 375                 380

Thr Ser Glu Lys Arg Pro Phe Met Cys Ala Tyr Pro Gly Cys Asn Lys
385                 390                 395                 400

Arg Tyr Phe Lys Leu Ser His Leu Gln Met His Ser Arg Lys His Thr
                405                 410                 415

Gly Glu Lys Pro Tyr Gln Cys Asp Phe Lys Asp Cys Glu Arg Arg Phe
            420                 425                 430

Ser Arg Ser Asp Gln Leu Lys Arg His Gln Arg Arg His Thr Gly Val
        435                 440                 445

Lys Pro Phe Gln Cys Lys Thr Cys Gln Arg Lys Phe Ser Arg Ser Asp
    450                 455                 460

His Leu Lys Thr His Thr Arg Thr His Thr Gly Lys Thr Ser Glu Lys
465                 470                 475                 480

Pro Phe Ser Cys Arg Trp Pro Ser Cys Gln Lys Lys Phe Ala Arg Ser
                485                 490                 495

Asp Glu Leu Val Arg His His Asn Met His Gln Arg Asn Met Thr Lys
            500                 505                 510

Leu Gln Leu Ala Leu
        515

<210>  3
<211>  21
<212>  DNA
<213>  Artificial Sequence

<220>
<223>  SEQ ID NO 3, forward primer

<400>  3
ctccagtgct cactctccct c                                                    21

<210>  4
<211>  21
<212>  DNA
<213>  Artificial Sequence

<220>
<223>  SEQ ID NO 4, backward primer

<400>  4
ccttagcagt gtgagagcct g                                                    21

SEQUENCE LISTING

<110> Medizinische Hochschule Hannover

<120> Prognostic marker for mamma carcinoma

<130> M1031EP

<140> EP10194061.7
<141> 2010-12-07

<150> EP10193883
<151> 2010-12-06

<160> 5

<170> BiSSAP 1.0

<210> 1
<211> 3029
<212> DNA
<213> Homo sapiens

<220>
<221> source
<222> 1..3029
<223> /mol_type="DNA"
      /organism="Homo sapiens"

<220>
<221> CDS
<222> 197..1750
<223> /codon_start=1
      /gene="exon 7"
      /note="SNP ID rs16754"
      /transl_table=1
      /translation="LQDPASTCVPEPASQHTLRSGPGCLQQPEQQGVRDPGGIWAKLGAAEASAE
      RLQGRRSRGASGSEPQQMGSDVRDLNALLPAVPSLGGGGGCALPVSGAAQWAPVLDFAPPGASAY
      GSLGGPAPPPAPPPPPPPPPHSFIKQEPSWGGAEPHEEQCLSAFTVHFSGQFTGTAGACRYGPFG
      PPPPSQASSGQARMFPNAPYLPSCLESQPAIRNQGYSTVTFDGTPSYGHTPSHHAAQFPNHSFKH
      EDPMGQQGSLGEQQYSVPPPVYGCHTPTDSCTGSQALLLRTPYSSDNLYQMTSQLECMTWNQMNL
      GATLKGVAAGSSSSVKWTEGQSNHSTGYESDNHTTPILCGAQYRIHTHGVFRGIQDVRRVPGVAP
      TLVRSASETSEKRPFMCAYPGCNKRYFKLSHLQMHSRKHTGEKPYQCDFKDCERRFSRSDQLKRH
      QRRHTGVKPFQCKTCQRKFSRSDHLKTHTRTHTGKTSEKPFSCRWPSCQKKFARSDELVRHHNMH
      QRNMTKLQLAL"

<400> 1
ccaggcagct ggggtaagga gttcaaggca gcgcccacac ccgggggctc tccgcaaccc      60

gaccgcctgt ccgctccccc acttcccgcc ctccctccca cctactcatt cacccaccca     120

cccacccaga gccgggacgg cagcccaggc gcccgggccc cgccgtctcc tcgccgcgat     180

cctggacttc ctcttgctgc aggacccggc ttccacgtgt gtcccggagc cggcgtctca     240

gcacacgctc cgctccgggc ctgggtgcct acagcagcca gagcagcagg gagtccggga     300

cccgggcggc atctgggcca agttaggcgc cgccgaggcc agcgctgaac gtctccaggg     360

ccggaggagc cgcggggcgt ccgggtctga gccgcagcaa atgggctccg acgtgcggga     420

cctgaacgcg ctgctgcccg ccgtcccctc cctgggtggc ggcggcggct gtgccctgcc     480

tgtgagcggc gcggcgcagt gggcgccggt gctggacttt gcgcccccgg gcgcttcggc     540

ttacgggtcg ttgggcggcc ccgcgccgcc accggctccg ccgccacccc cgccgccgcc     600

gcctcactcc ttcatcaaac aggagccgag ctggggcggc gcggagccgc acgaggagca     660

gtgcctgagc gccttcactg tccacttttc cggccagttc actggcacag ccggagcctg 720
tcgctacggg cccttcggtc ctcctccgcc cagccaggcg tcatccggcc aggccaggat 780
gtttcctaac gcgccctacc tgcccagctg cctcgagagc cagcccgcta ttcgcaatca 840
gggttacagc acggtcacct tcgacgggac gcccagctac ggtcacacgc cctcgcacca 900
tgcggcgcag ttccccaacc actcattcaa gcatgaggat cccatgggcc agcagggctc 960
gctgggtgag cagcagtact cggtgccgcc cccggtctat ggctgccaca cccccaccga 1020
cagctgcacc ggcagccagg ctttgctgct gaggacgccc tacagcagtg acaatttata 1080
ccaaatgaca tcccagcttg aatgcatgac ctggaatcag atgaacttag gagccacctt 1140
aaagggagtt gctgctggga gctccagctc agtgaaatgg acagaagggc agagcaacca 1200
cagcacaggg tacgagagcg ataaccacac aacgcccatc ctctgcggag cccaatacag 1260
aatacacacg cacggtgtct tcagaggcat tcaggatgtg cgacgtgtgc ctggagtagc 1320
cccgactctt gtacggtcgg catctgagac cagtgagaaa cgccccttca tgtgtgctta 1380
cccaggctgc aataagagat attttaagct gtcccactta cagatgcaca gcaggaagca 1440
cactggtgag aaaccatacc agtgtgactt caaggactgt gaacgaaggt tttctcgttc 1500
agaccagctc aaaagacacc aaaggagaca tacaggtgtg aaaccattcc agtgtaaaac 1560
ttgtcagcga aagttctccc ggtccgacca cctgaagacc cacaccagga ctcatacagg 1620
taaaacaagt gaaaagccct tcagctgtcg gtggccaagt tgtcagaaaa agtttgcccg 1680
gtcagatgaa ttagtccgcc atcacaacat gcatcagaga aacatgacca aactccagct 1740
ggcgctttga ggggtctccc tcggggaccg ttcagtgtcc caggcagcac agtgtgtgaa 1800
ctgctttcaa gtctgactct ccactcctcc tcactaaaaa ggaaacttca gttgatcttc 1860
ttcatccaac ttccaagaca agataccggt gcttctggaa actaccaggt gtgcctggaa 1920
gagttggtct ctgccctgcc tacttttagt tgactcacag gccctggaga agcagctaac 1980
aatgtctggt tagttaaaag cccattgcca tttggtgtgg attttctact gtaagaagag 2040
ccatagctga tcatgtcccc ctgacccttc ccttcttttt ttatgctcgt tttcgctggg 2100
gatggaatta ttgtaccatt ttctatcatg gaatatttat aggccagggc atgtgtatgt 2160
gtctgctaat gtaaactttg tcatggtttc catttactaa cagcaacagc aagaaataaa 2220
tcagagagca aggcatcggg ggtgaatctt gtctaacatt cccgaggtca gccaggctgc 2280
taacctggaa agcaggatgt agttctgcca ggcaactttt aaagctcatg catttcaagc 2340
agctgaagaa aaaatcagaa ctaaccagta cctctgtata gaaatctaaa agaattttac 2400
cattcagtta attcaatgtg aacactggca cactgctctt aagaaactat gaagatctga 2460
gatttttttg tgtatgtttt tgactctttt gagtggtaat catatgtgtc tttatagatg 2520
tacatacctc cttgcacaaa tggaggggaa ttcattttca tcactgggag tgtccttagt 2580
gtataaaaac catgctggta tatggcttca agttgtaaaa atgaaagtga ctttaaaaga 2640
aaatagggga tggtccagga tctccactga taagactgtt tttaagtaac ttaaggacct 2700
ttgggtctac aagtatatgt gaaaaaaatg agacttactg ggtgaggaaa tccattgttt 2760

aaagatggtc gtgtgtgtgt gtgtgtgtgt gtgtgtgtgt gtgttgtgtt gtgttttgtt 2820

ttttaaggga gggaatttat tatttaccgt tgcttgaaat tactgtgtaa atatatgtct 2880

gataatgatt tgctctttga caactaaaat taggactgta taagtactag atgcatcact 2940

gggtgttgat cttacaagat attgatgata acacttaaaa ttgtaacctg catttttcac 3000

tttgctctca attaaagtct attcaaaag 3029

<210> 2
<211> 517
<212> PRT
<213> Homo sapiens

<220>
<221> SOURCE
<222> 1..517
<223> /mol_type="protein"
/note="This sequence is generated from feature [CDS]:197..1750 pl
aced under sequence number 1"
/organism="Homo sapiens"

<400> 2
Leu Gln Asp Pro Ala Ser Thr Cys Val Pro Glu Pro Ala Ser Gln His
1               5                   10                  15
Thr Leu Arg Ser Gly Pro Gly Cys Leu Gln Gln Pro Glu Gln Gln Gly
            20                  25                  30
Val Arg Asp Pro Gly Gly Ile Trp Ala Lys Leu Gly Ala Ala Glu Ala
        35                  40                  45
Ser Ala Glu Arg Leu Gln Gly Arg Arg Ser Arg Gly Ala Ser Gly Ser
    50                  55                  60
Glu Pro Gln Gln Met Gly Ser Asp Val Arg Asp Leu Asn Ala Leu Leu
65                  70                  75                  80
Pro Ala Val Pro Ser Leu Gly Gly Gly Gly Gly Cys Ala Leu Pro Val
                85                  90                  95
Ser Gly Ala Ala Gln Trp Ala Pro Val Leu Asp Phe Ala Pro Pro Gly
            100                 105                 110
Ala Ser Ala Tyr Gly Ser Leu Gly Gly Pro Ala Pro Pro Pro Ala Pro
        115                 120                 125
Pro Pro Pro Pro Pro Pro Pro Pro His Ser Phe Ile Lys Gln Glu Pro
    130                 135                 140
Ser Trp Gly Gly Ala Glu Pro His Glu Glu Gln Cys Leu Ser Ala Phe
145                 150                 155                 160
Thr Val His Phe Ser Gly Gln Phe Thr Gly Thr Ala Gly Ala Cys Arg
                165                 170                 175
Tyr Gly Pro Phe Gly Pro Pro Pro Pro Ser Gln Ala Ser Ser Gly Gln
            180                 185                 190
Ala Arg Met Phe Pro Asn Ala Pro Tyr Leu Pro Ser Cys Leu Glu Ser
        195                 200                 205
Gln Pro Ala Ile Arg Asn Gln Gly Tyr Ser Thr Val Thr Phe Asp Gly
    210                 215                 220
Thr Pro Ser Tyr Gly His Thr Pro Ser His His Ala Ala Gln Phe Pro
225                 230                 235                 240
Asn His Ser Phe Lys His Glu Asp Pro Met Gly Gln Gln Gly Ser Leu
                245                 250                 255
Gly Glu Gln Gln Tyr Ser Val Pro Pro Pro Val Tyr Gly Cys His Thr
            260                 265                 270
Pro Thr Asp Ser Cys Thr Gly Ser Gln Ala Leu Leu Leu Arg Thr Pro
        275                 280                 285
Tyr Ser Ser Asp Asn Leu Tyr Gln Met Thr Ser Gln Leu Glu Cys Met
    290                 295                 300
Thr Trp Asn Gln Met Asn Leu Gly Ala Thr Leu Lys Gly Val Ala Ala
305                 310                 315                 320
Gly Ser Ser Ser Ser Val Lys Trp Thr Glu Gly Gln Ser Asn His Ser
                325                 330                 335
Thr Gly Tyr Glu Ser Asp Asn His Thr Thr Pro Ile Leu Cys Gly Ala

Gln Tyr Arg Ile His Thr His Gly Val Phe Arg Gly Ile Gln Asp Val
340 345 350
Arg Arg Val Pro Gly Val Ala Pro Thr Leu Val Arg Ser Ala Ser Glu
355 360 365
Thr Ser Glu Lys Arg Pro Phe Met Cys Ala Tyr Pro Gly Cys Asn Lys
370 375 380
Arg Tyr Phe Lys Leu Ser His Leu Gln Met His Ser Arg Lys His Thr
385 390 395 400
Gly Glu Lys Pro Tyr Gln Cys Asp Phe Lys Asp Cys Glu Arg Arg Phe
405 410 415
Ser Arg Ser Asp Gln Leu Lys Arg His Gln Arg Arg His Thr Gly Val
420 425 430
Lys Pro Phe Gln Cys Lys Thr Cys Gln Arg Lys Phe Ser Arg Ser Asp
435 440 445
His Leu Lys Thr His Thr Arg Thr His Thr Gly Lys Thr Ser Glu Lys
450 455 460
Pro Phe Ser Cys Arg Trp Pro Ser Cys Gln Lys Lys Phe Ala Arg Ser
465 470 475 480
Asp Glu Leu Val Arg His His Asn Met His Gln Arg Asn Met Thr Lys
485 490 495
Leu Gln Leu Ala Leu
500 505 510
515

<210> 3
<211> 21
<212> DNA
<213> artificial sequences

<220>
<221> source
<222> 1..21
<223> /PCR_primers="[fwd_name: SEQ ID NO 3]"
/mol_type="DNA"
/note="SEQ ID NO 3, forward primer"
/organism="artificial sequences"

<400> 3
ctccagtgct cactctccct c 21

<210> 4
<211> 21
<212> DNA
<213> artificial sequences

<220>
<221> source
<222> 1..21
<223> /PCR_primers="[rev_name: SEQ ID NO 4]"
/mol_type="DNA"
/note="SEQ ID NO 4, backward primer"
/organism="artificial sequences"

<400> 4
ccttagcagt gtgagagcct g 21

<210> 5
<211> 33133
<212> DNA
<213> Homo sapiens

<220>
<221> source
<222> 1..33133
<223> /mol_type="DNA"
/note="chromosome 11: 32341389..32374521"
/organism="Homo sapiens"

<220>
<221> gene
<222> 1..33133
<223> /gene="nucleotides 32341389..32374521 of chromosome 11"

<220>
<221> misc_feature
<222> 33133
<223> /standard_name="rs16754"

<220>
<221> misc_feature
<222> 32857
<223> /standard_name="rs5030277"

<220>
<221> misc_feature
<222> 32063
<223> /standard_name="rs5030281"

<220>
<221> misc_feature
<222> 26428
<223> /standard_name="rs5030310"

<220>
<221> misc_feature
<222> 32620
<223> /standard_name="rs5030280"

<220>
<221> misc_feature
<222> 10186
<223> /standard_name="rs11031755"

<220>
<221> misc_feature
<222> 2409
<223> /standard_name="rs11031747"

<220>
<221> misc_feature
<222> 2102
<223> /standard_name="rs3858439"

<220>
<221> misc_feature
<222> 1742
<223> /standard_name="rs3858438"

<220>
<221> misc_feature
<222> 1607
<223> /standard_name="rs11031746"

<220>
<221> misc_feature
<222> 1507
<223> /standard_name="rs11031745"

<220>
<221> misc_feature
<222> 1427
<223> /standard_name="rs11031744"

<220>
<221> misc_feature
<222> 765

<223> /standard_name="rs3858436"

<220>
<221> misc_feature
<222> 1
<223> /standard_name="rs2418901"

<400> 5
attacattta gagtccacct ccagaatcca ggaaaatctc ccaatcttaa aatccttagc 60

ttaatcacat cagcaaagtc ccctttgcct tgtatcctgg gttctaggga ttaggacatg 120

gacatctgtg ggaagacatt aatctgccta ctacagggga tttgtctaga ggtgctgtgg 180

taagccctac cacaggagaa atgtagggtg tatgagagtt tgcaggaatg ccagatttgg 240

cgatcaggaa aagctcctct gtttagtctg agacctggag gatgaataag aaggatttgg 300

gaggccgggt gtggtggctc atgcttgtaa tcccagctac ttgggaggct gaggcaggag 360

aatcacttga acccaagagg tgaggttgca gtgagctgag atcgagccac tgcattccag 420

cctgggcgac agagcaagcc tctgcctcaa aaaaaaaaaa aaaaaaaaaa gaaggatttg 480

ggagacatga ggaagaggaa gttatactga aagaattcag atagtttaga acaaaggctt 540

aaaagtcaca gagaatgaag tagatctttg gagctgggag tcactgagca agttgttaca 600

aggctgcaag gtaaggacca ggcatgaaag gcctgggaag ccctgggaag tttaggcttt 660

aacctaaggg caaagggtgg caaggtgaga tggaaaagtc acttggctcc tatgccagtc 720

cagattgaag ggctggtgat ggagagaata gccaggaggt atgatagtaa tccagagaca 780

gatgctggga ctaggagggc tggggacagg tggaggcatg aggagagatt gagatttatt 840

tatttgtttt attattatta ttctttgaga cacagtctcc ctctgtcgcc caggctggag 900

tgcagtggca ctatctcagc tcactgcaac ctctgcctcc ctggttggag caattcttat 960

gcctcagcct cctgagtaat cctcagctgg gattacaggc gtgcgtcacc atgcccggct 1020

aatttttttg tatttttaat agagatgggg tttcaccatg tttcccaggc tggtctcaaa 1080

ctcctgggct caagtgatcc acctgccttg gcctcccaaa gtgcggggat tacaggcgtg 1140

agccactgca cccagatgag attgagactt agatttagga ctgtcagggg tcggcagttg 1200

gctggatgtt gggtgaggat tacagaagga ggagtcccag gtttctggct tgagctacat 1260

ggggaagaag gccatggttc ctgttttgaa catgttgtct tggtgcctag gagagatcgg 1320

agtggactgc ctaggaaaca gctggctgca gctctgtctt ggggtctggt gaggattaaa 1380

caaatgaagt atggaaagca cttagcccag tggctggcac atagtccgta cttaataaat 1440

atgaaatatt attattatgt attagtttta tattcaggtc tgtgctgtac cttatcaaat 1500

tatgccctga gagccatttg gagcaaaata aaaagaaggc aagtaaatga caagagagca 1560

aggaaaaggg tggttctgta gctgcacacg tatctgtgtt ctcacgagag tcccacaggc 1620

taacagggaa aggatggggc agggcttgcc tgttaaagtg gaggcatcag ttagcatgaa 1680

ccaaatacag atggggaaaa taactaccct gagtctgctc actcctgcaa ttctaggatc 1740

agacatccca ggtgaggagt ccccatgaag cggcatcgtt tgtctggggt aatacctgag 1800

gttggttgcc tcatgccaag gaaatcaagg acacggacac acgtggagtg aagattaaga 1860

atggaggttt aataggcgaa attaagaaaa aagagaaata gctctccctt ctgcagagag 1920
agaggagatc ccgagggggt cttctggttt ggtggtgaaa tgcacagggt tttatagtct 1980
ggtttgcgga ggctgtgtct gacttacata gggcccaaag attggttgga ccaggcatga 2040
catttataca gcccgtgaag aagttggcca ccccacccta atcttttgtt atgcaaatga 2100
gtttttttaa ctggccagca ccacattgtc agctccttac tgtacacatg gttgataaag 2160
gaaagggaag atggagcctc catgttggac atgtctagcc cccaggtagc cttttcctat 2220
tgtcacagct gcaggcattc acctctgcaa gcttccgact tgcttatcta tgtttgcagc 2280
tcaattttac aggctgcttt ttgttagaaa agaaatgatt tggccgggcg cagtggctca 2340
tgcctgtaat cccagcactt tgggaggccg aggcaggcgg atcacctgag gtcaggagtt 2400
caaggccctg tctctctact aaaaatacaa aaattagccg ggcgtggggg ccggcacctg 2460
taattccagc tacttggagg ctgaggcagg agaatcgctt gaatccggaa ggcggaggtt 2520
gcagtgagcc gagatggcgc cactgcactt cagtctaggc aacaagagcg aaactccgtc 2580
tcaaaaaaaa aaaaaaaaaa acactggggg gctgctcttt atgaaaaggg aaaccttacc 2640
aaggacttcc ttatcctcac tatctgtcta aataatttct tcttaactcc tgtatcaccc 2700
agatatctcc gtgaggagtc aggagggaaa tttgggctga ttgctgggtg tttttgtttt 2760
tgtttgtttt tggttttttt tgagacggag tctcgggcag ctacccaggc tggagtgcag 2820
tggtgcaatc tcggctcact gcaagctccg cctcctgggt tcacgccatt cttctgcctc 2880
agcctccgga gtagcaggga ctacaggcat ccgccaccac gcctggctaa ttttttgtgt 2940
ttttagtaga gacggggttt caccgtgtta gccaagatgg tctctatcta ctgacctcgt 3000
gatccgcccc cctcagcctc ccaaagtgct ggaattacag gcatgagcca ccgaccccgg 3060
cccaggctgg tttgttttta atgagaggct gggtggctgt tagggagggg gacgtccttt 3120
ctcgtcaata tcaacaatgt cagcaacatc agcaatgaac tgatttttct cttcactctg 3180
atttcaaccc tagtattgcc atcaaagaga atcctccgtc tgcacaactg caaacacggg 3240
ccttaaaaga aggctggggc aatttctcca agtggtttca tattagtact tgccccttag 3300
agagaagaat tgattttcct gggcaaagta acatttggta gaaggaaaag ttttatgatt 3360
tatgatgcac cagagaccat atatttccct taaatattat cattatggtc atagcacttc 3420
aaaagagaaa aatggaattc aaggaacatt aacttctaaa tatttacggc atttcaaatc 3480
cctgtccagt cttcattgtc actaggataa ctttgagaag ttgtgagcac atagacaatg 3540
caatgaaaaa tagccagaat ccagctgagt gttgtgttca tatccttctg tggttgttgt 3600
ggggctccaa gactggaaaa atatgaaaag aatgcaggat tccagcaagt acaaaggtct 3660
ttagaactag accagaaaca tgtaaatatc ttgagtcaaa gaaatgcaca attagtcaag 3720
tgaagtcaca gattgggaag tgtgcaggct tctctgagta gagaaaaggc tggggacttg 3780
gctgtaagac cacccccatt tggaaaccat aagatataga accgtaaata aagctcagtg 3840
ggtctttcag tcttgtggac agcattgcag tcagcactca gattcaactg tgacaaaggt 3900

aaccccaaaa gtgttcagga taatgaaatt ttttcctcag aaggtaaaaa gttccttaat 3960
tctgaaacct ggggtatttt ttatacataa caatagaatg gcaagagaat aaaacaaaaa 4020
tattagcatt tttatgttaa gacatctgag catatttgat attttgagtg taaggtttaa 4080
gaaagttggg ttggctgggt gtggtggctc acacctgtaa tcccagcact ttgggaggct 4140
gaggtgggca gatcacgagg tcaagagatc aagaccatcc tgaccaatat gatgaaaccc 4200
catctttatt aaaaagtaca aaaattagct gggcattgtg gtgcatgctt gtagtcccag 4260
ctacttggga ggctgaggca ggagaatcgc tagaacccag gaggcagagg ttacagtgag 4320
ccaagatcgt gccactgcac tccagcctgg gcaacagggt gagacttcat ctcaaaaaaa 4380
tttaaaaaaa ggaaagaagg ttgggtctat tagagttgat aagtcagtct tgaaattcta 4440
ttttaaaatg taatgagtga ttaatgtgta tgtgtaatat ttaaactaaa ttctgattaa 4500
atttacatac caggttgaaa agggcttgag aattactata ttagtctttt cacactgctg 4560
ataaagacat gtgtaattta aaaggaaaaa gaggaaatat attaatattc atacatgttg 4620
cacctatcct tctattatcc ttgaacccta aaattacaag actgtgtaat ttaaaaagaa 4680
aaagaggttt aatggactca tggttccact tggctgggga ggcctcacaa tcgtggttga 4740
aggtgaaagg cacacggtgg cagacaaaag agaatactag aacttgtgca gggaaattct 4800
cttttataaa actatcagat cttgtgagac ttattcacta tcatgaatac agtacaggaa 4860
agacccgccc tcatgattca attatctccc acttagtcct tcccatgaca catgggaatt 4920
gtgggagcta tgatgcaaga tgggatttgg gtggggacac agccaaacca tatcattctg 4980
cccatggccc cttccaaatc tcatatcttc acatttcaaa accaatcatg ccttcctgat 5040
ggtcccccaa agtcttaact catttcagca ttaactgaaa agtccacagt ccaaagtctc 5100
aactgagaca aggcaaatcc cttctgccta cgagcctgaa aaaatcaaaa gtaagttagt 5160
tacttcctag ataaaatggg ggtacaggca ttggataaat atacccattc caagtagaaa 5220
aaattggcca aaatgaaggg gctaaaggcc ccatgcaagt ctgaaatcta gtggggaggt 5280
caaatcttaa agctccaaaa aaaaaaaaaa aagatctcct tttactccat gtctcatatc 5340
caggtcacgc tgatgcaaga ggtgggttcc catggtcttg ggcagctctg cccctgtgcc 5400
tttgcaaggt acagcctccc tcctggctgc tttcacaggc tggtgttgag tatctatggc 5460
ttttccaggt gcgtggtgca agctgttggt ggatctacca ttctcaggtc tggagaacgg 5520
tgaccctctt ctcacagctc cattaggcag caccccagtg gggactctgt gtggggttgc 5580
ccaccccaca tttcccttct gcactgccct agcagaggtt ctctatgagg gccccacccc 5640
tgcagcaaac ttctgcctgg atatccaggc atttccatac atcctctgaa atctaggcag 5700
aggttcccaa acctcacctc ttggcttctg tgcacctcca ggctcaacac cacgtggaag 5760
ctgccaagac ttggggcttg caccctctga agccacagcc tgagctgtac cttggcccct 5820
tttagtaatg gctggagcag ctgggacaca gggcaccaag tccctagact gcacacagca 5880
gtgtgaccct gggcccagcc cacaaaacca ttattccttc ctaggcctcc aggcctgtga 5940
taagaggggc tgctgcaaag ttctctgaca caccctggag acattttccc ccattatctt 6000

ggtgattaac attcagctcc tccttactta tgcagatttc tgcagccggc ttaaatttct 6060
cctcagaaaa taggattttc ttttctgttt tctctatcag gctgcaaatt ttctaaacct 6120
ttatggtgtt tcccttttaa aattgaatgt ttttaacact tcttgaatgc tttgctgctt 6180
agaaatttgt tctgctagat accctaaatc atctctctca agttcaaagc tccacaaatc 6240
tccagggcag gggcaaaacg ccaccagtct ctttgctaaa gcataacaag agtcaccttt 6300
tctccagttc ccaacaagtt cctcatttcc atctgagact acttcagcct ggattacatt 6360
gtccacatca ttatcagcat tttggtcaaa gccattcagc aagtctccag ggagttccaa 6420
actttcccac attttcctat cttcttctga gccctccaaa ctgttccaac ctctgcctgt 6480
tacccagttc caaagtcact tccacatttt caggtatctt ttcagcagcg ccccattcta 6540
ctggtaccaa tttaccatat tagtcagttt ttatgctgct gataaggaca tacccaagac 6600
tgtgtaattt aaaaagaaaa agaggtttaa tggactcaca gttccatgtg gctggggagg 6660
cctcacaatc atggtggaag gcaaaaggca catcgtacac agcagcagac aagagagaat 6720
aatagaactt gtgcagggaa actccccttt ataaaactat cagatctcat gagacttatt 6780
cactatcatg agaacagcat aggaaagacc tgcccccatg attcaattac ctcccactgg 6840
gcccctccca tgacacatgg gaattgtagg agctacagtt caagatgagt tttgggtggg 6900
gacacagcaa aaccatatca atcaccatat ttatgagttt agtttactag aatttaaagt 6960
gttgtttagg ttcccaggaa agttttgttt gttgactcag actggtataa ggaaggaagg 7020
aaggaaggaa ggaaggaagg aaggaaggaa ggaaggaagg aaggaaggaa ggaaagaagg 7080
aaagaaagaa agaaggaaag aaagaaagaa ggaaacaaag agagcagtaa ggacaagaga 7140
catccttgtc tgcttctgtt ttgaaggaga attgctttgg ttcttctaat attggctttt 7200
gcatttaatt ttttaaatct tttttttttg agataggttc tcactctctt gcccaggctg 7260
gagtgcagtg gtgtaatcat agctcactgc aggctcaaac tcctgggctc aagggatcct 7320
cccacctcag cctcccggta gctgggacta caggcatgtg cccccattcc caactaactt 7380
tttaattttt gtagagatag ggtctcacta tgtttcctag gttgatctcc aacttctgac 7440
ctcaagccat cctcctgcct tggcctccca aagtgctgga attacaagca taagccacct 7500
tgcttggcct aatttctaaa actttatatt atggaaattt ttaacatgca caaagataag 7560
acagaaaagt acactattaa acccccatgt ggtcactact cagccttcac aattttttaa 7620
catttctcca ttcctgtttt attcattctt gttttataac cccactccaa acattctttt 7680
ctcctcctcc ttctcctttt tttgctttga ggtcgtggtc atgaggcaga ctggaaggtt 7740
ttttttttta actgaggtaa aaatcaatga aatgtacaga ttttaagtgt acagtttgat 7800
gactttcatt aaatgtatat gcccaagtac tcaacaatcc aatcgagata aagaacattt 7860
cattacccta gaaaatttcc ctgtgccctc ttccaggaaa acatcaccct ccacacacaa 7920
ccacttttct gatttatatc atcatagaaa tggtgccttt tcttgagctt catatattag 7980
gaatgacgtg gtatcttccc ctttgtctga cttctttcat tcaaactttt ttgggattca 8040

tccatgttgt tgcatatatc agttgttcat ttttcaaatt gctggatagt attgcattgt 8100
atgaatgtgc cacactttat tcacttatac cttctcccat tgatagacat ttgggttgtt 8160
tgcaattttt agctattctg aatacaagta atataaacat tcttatacaa ttcttttttc 8220
tcattcttgt acaattctta ttttggatat ttgttttcat ttttgtgggg gtagataaca 8280
aagaatatag aattgcttgg ccatggggta gatgtatact tagctttata tggaactgcc 8340
aaacagtttt tccaaaatgg ctttgctatt tcacactctc ctcagcagtg tatgagaagt 8400
ctagctgttg cacatccttg gcggtatttg gtgttgtcag ttttttccac tggagccatt 8460
tcagcaaatg tgaaatggca tctcattgtt gttttaatgt gcacattgct atggtctgaa 8520
tgtgtccccc caaatttcat atgttgaaat cctgaccccc aaggtgatgg tattaggatg 8580
tggggccttt gggagatgat taggccatga ggacagagcc cttgtgaatg ggattagtgc 8640
ccttagagaa gaggtcctaa agagtgaatt ctttccctcc accatgtaag gacacagcaa 8700
ggggccatct atgagccaga gagcagccct caccagacac caggcctgca ccttgatgtt 8760
ggacttccca gcctcatgaa ctgtgacgaa taaatttctg atgtttataa accatctagt 8820
tatgttatct tgctatatag cagggtgaat agactaagac acaagtgtaa tgtgtgtgtg 8880
tacagaaatt cttcctacca aattttaaag agtttaatta agaaaagtga taagtttttt 8940
gtggaagatg tggtgtttta tacttattaa gataattatt tttcttttta aatagtaaac 9000
cattaataat ttatatgaat gataccttcc attcccaaaa taaacctttt ttgaaaggaa 9060
tgaagtgatg acgcatgctg accacagatg aaacttgaaa catgctaagt ggaagtaaca 9120
atgactctct ccttggccaa actttaatca gtgtcctcta agcccttttc tcaactaagc 9180
tcaggtttgc agagcccaga ttgagcaaaa atcctattaa gtccagtgtg tgtgtgtgtg 9240
tctgtgtgtg tgtgtgtgtt tgtgtgtgtg tgtgtaattt tttgtgacgg agtctcactg 9300
tgttagctag gctgctcttg aactcctgga ctcaagcaat cctcccacct cggcctcctg 9360
agtagctggg attacaggtg cacacccaaa ataaacctta tatacccata gtggacaata 9420
tttttttgag acagggtctc attgtgttgc ccatgctgga gtgcaatggc acgatctcag 9480
ctcactgcaa cctccacctc ccaggttcaa gccattctcc cacctcagcc tccccagtag 9540
ctgggactgc aggtgcacac caccacgccc agctaatttt tgtatttttt ggtagaggca 9600
ggggtttcac catgttggcc aggctggtct cgaactcctg acctcagatg atccgcccac 9660
ctcagcttcc caaagtgctg ggattatagg cgtgagccac catgactggc cacttctttt 9720
aatgtgttgt tttattctaa gtcatacaat ctgatatacc attttttaat cgtttcccac 9780
gtatgcttag tttaaattat ttggtggggg aaagggcagg gtagaggcag tattgtatcc 9840
ttgtcagatt caaggatcaa aaacaaattc aatttataca atcatctcag ttaacaggcc 9900
attccttttc ccgatatttt gagagaattc atgtaataaa gggattattt atagtgccgt 9960
cgaaagagca ttgacttggg aggaaaacag aactgctctt gatgtgagtt ggccactcac 10020
cagccttatg acctagcaaa ttatttcatc tctctttcag tatcttccac atcactgagc 10080
aagtggatgg agagaggaaa gacacctggt gaatcccggt gataagactg atgattggaa 10140

ataaaatttg ccatgaaatg ggacaagcat actccagtat gactgcatca caattaattg 10200
catctgcaat gaccttattc ccaaataaag tcacattctg aggtgacgtg aattttggaa 10260
tgacactatc cagaccacac acctatattc attcatattt ggaaactcca tttcgggtta 10320
tgaaacacgt gcctcacttg ttgcatgccc tttgcagaca gggtgatgca aagtgtgaca 10380
cagtgtgacc tgggttccag tcattgtcct gccttgtgct agatgaccct gggcaatcta 10440
attcatcttt ctaggcctca tccaaataat gaagtcagga gaggtggtct ctaagaaccc 10500
ttggaattta attattcaca agcttttggg actcaattaa acattttttt tctttctttt 10560
tttttttttt tttttttttt ttttcagggg aaatgtagga atgagccatc atgaaacgat 10620
gccagaggct ggagagcctg gatacattga cacaattggg ggatgaatga tttattacaa 10680
ttcaattcca gggcagccca gtctacagct ccccaggaag catctctaaa ggtgctactt 10740
gttaatcatc accatacgaa aggcgggggg gattgctttc atctgtggct tttatgagac 10800
catccagcta ttcttcagag ccagtttctc atcaaaaatg gggtttatta ctacaaaagt 10860
taaagacctt agtacacatt ttggtaaaca tagattgata tcccctagca ggaatcaaga 10920
gaacagattt catttactga aaggttacca ggcattgtgc taagggctct acatggcttc 10980
tttccattta atcccccaaa caactctatg aagtcgtgtg ccaggtaatc aatgtatcct 11040
tctcaggtcc aaattcaccc ttcagtacct gctctgccat aatgacgtgt aaagcatttg 11100
tcctttacat tgagcgccac gataagcttt gtcagtagag ggcgctggag agacatcacg 11160
ggaggacgcc gcttctcttc ctggttctgg cccgctcttt tgcttggttt gctccacctg 11220
ctttgtaaag cgtcctcctg tgcatatgtg aggacatcca gtggcattct gctccaccca 11280
ggctcccaga gtgcccagtc cctcagccac tttgcagccc tgctgggggc ccagtgacca 11340
ccttgctgtg gccttcctgt tgcaaatgcc agtgcacccc agggctcccc aacacgctcc 11400
cggcctaccc tccagcctcg gtttgcctgc ctccctgggg agggctgttt tccaccgccc 11460
tactaacatc aacaccacga atcctaggcc aattgtcgcc ctgccaggaa gcagattctc 11520
agcatcctga ctccatctgc ccactcacct tggctcacct ggacctggga gggctgtttc 11580
ctgctaccag gaagcccaca gacttcacca tccagtgggt ggcaccatgc cttctccaac 11640
ctagtccaaa ccccagcctc ggggaaggcc tcccccttcc aagtttgtcc tttcttgggc 11700
actttccctc caccctggag gacccttaga gttcccttca catcttcatg gatactcacc 11760
tatcatcatt tcatacttct ttagattaaa acaatccctg ttcaaattgc tgtgtggttt 11820
ctgtctcctg attggatgca gacagataca agtaggtact attatttcca ttttaaagat 11880
gagcgaatga gatttaaaga ggccaaatgg cttagtgaga atcagatggg aaatgtggca 11940
gagctggaca caaaccccag gatggtctgg ctctgaagcc tttgatttgc acctctctgt 12000
acataaacca ggagttagtg gacagatacc acataagaaa gaggggtgat ccagccttct 12060
ccctcccctc ttttcctatg cttatgtgtc ctgggtgttt cagatgaaaa taagggcaaa 12120
aaatgcaaaa atgggccggc gcagtggctc atgcctgtaa tcccaacact ttgggaggct 12180

gaggtaggtg gatcacttga ggtcaggagt tcaagaccag cctagccaac atggcgaaac 12240
cccatctcta ctaaaaaata caaaaattag ctgggtgtgg tagagggtgc tggtaatccc 12300
aactactcgg gaggctgagg caggagaatc acttgaacct gggaggcaga ggttgcagtg 12360
agctgagatc gcaccactac actccagcta ggcaacagag tgagacttcg tctccaaaaa 12420
aaaaaaaaaa aaagaaaaag aaaaagaaaa aaatacaaaa atgaggccag ctcctagagg 12480
aagattttca acgatcaaga atgtcaatag tcatctcaag ataattgcca aaataaacac 12540
agaaaagaag gggagaaaaa aaatgcccca tgtatttaaa aaaccacttt tatacacttt 12600
acatatggtc aatactctgc tccaaataca tttgttttga ggctattcaa tttttgccaa 12660
tttccccagc tttgtcttat ctgctaaaat gcctcatcct gctatggaga gaggtgaaat 12720
cccagctttg ggtgttgcct gtatttgcat ctggctcaaa gcagccagat ttctggatga 12780
tgtatgtccc agctctaagg ggctccggtc cccactgggg gtgtgctcag aggcagcatg 12840
taacagcttc catattcctg gaaatgaagc cccaacttcc ttgtagttta tcctttaggg 12900
aaatcagcaa tgtgaatcct aattagcctc aaaataacaa accccagtaa ttaatgatct 12960
tccttggggg atcattgttc tggactttca gcttcagtag ggaggggaaa aaaaaggtcc 13020
aataggagga ttcaccattt cctgctgatt agagctggtg agctctggac tcagagttag 13080
gatacctgga tttgagtcct ggctttgcca ctaattacac ggtcctttgt ctctctgtaa 13140
acaaaaaaga ttggcatcta agctcctttc tagttttgtg gtccttcagc ctcagaatcc 13200
tttcaactct atttagccat gccaagttaa ataacagcac cttcactgag tgctcacaat 13260
gtgccaggca aattgctaaa caccttaaat acattatcta atttaagcct cactgcaaac 13320
ttatgaggtg agcactatta ttattatttt tttattttat tttttgagat ggattctcac 13380
tgtcaccagg ctggagtgca gtggtgcgat ctcggctcac tgcaacctct gtctcccagg 13440
ttcaagcgat tctcctgcct cagcctcccg agtagctggg actacaggca tgatccacca 13500
tgcccagcta atttttgtat gtttagtagg gacggggttt cactatgttg gccagggttg 13560
tcttgatctc ttgacgtcat gatccgcccg ccttggcctc ccaaagtgct gggattacag 13620
gcgtgagcca ccgtgcccag cctattatta tccccatttt acaggttcag agagattaga 13680
gcatgatgat aagaaagcca caccatgtgt cctctcccca gatgggttac tgaccttaca 13740
aatatgctcc aaatggggtc cagtaggaga aatgcaggta gatccctgat tgacattgtt 13800
caagtcagca tcaccctcat tagcggcaaa tgaggataag cagtaataag tagtcaggca 13860
ggtgcttgga gccaaaggca ggagggaaaa ggacattaat tagcaaagcc aggcacttta 13920
tgcaggtggt ctcatttaat ccttagagca atgctataca ttgggattat tcccattata 13980
cagataaaga aaatgaggtt cagagaggtt aagtgacttg ttcacttaca cttcccacca 14040
ccggtcagca gccaacctga gattgcagtg tattttgttt gacctctttc atcacactgc 14100
attgggacaa tccattgttt gcggccaggg atttcagggt ctgagcaacc atacatacat 14160
ttattgaggc caagaccatg tcaactgaca gtgaaataac tgttcaattc agacaaaggg 14220
aaggtgggga aaggtcttta aaaagttagt ccttttcttt gagattggct aagctgagat 14280

cccctgggtc tacgcccagc ctctgcccca gcccctccag gacaagtgct gtcctgcctc 14340
tgagagaaga aaccaaggac ctgctaatca tctccatggg aactgaccca ggggttggac 14400
ctcaaggagg cttcagaata ggccagttgg aggaagaaca ggaacaggct gtgaggcaga 14460
aacaaggaac aaacctagcc atcctcaaag aacaagaaaa agtcacattt tttttctatt 14520
tctcttttga ccctatcttt gaagactaca tgcttaagaa gaaccactcc gctggacctt 14580
ccagacacaa ggaataattg agcacataga gacacgttgc tcaggtgctc ttgggatata 14640
acttctcagc caccatccaa agagtagcct accatggtat acaggtaaac tggttctctg 14700
gaaaacaaag caacaaaggc agtcctgctt tgtagcactt gccaattccc atggtgtaaa 14760
gactttcagt tcaagctact aacactttaa catttggctt gcaaaatacc taaatattta 14820
acaactgcct tcagctaaac aagaagtgac atcttggtta gaaaacatgt cttatgggcc 14880
aggcacggtg gctcatgcct gtaatcccag cactttggag gccgaggtgg gtgggagcca 14940
gttgttaaag agttagcagc tcagctgcta ctagcaaacc agtggaaacc agttccatgt 15000
accctgggta gccagctttg agggtacacc attctgagca agcaagagtc tcttgtggac 15060
aatggcccag tattctcctt ccccactgtg actccaggta tgccaagata ttccctgtcc 15120
ttttaattcc tgcacatcct tttacctgga agcttcttcc ctttctcttc cttctgaaaa 15180
actcctatac atccttcaag aaccaggctg aaaataactc tgttaaacct tccccctgtt 15240
aaagctcatt gttgatcctt tgcccatgcc tgtattacct gtttaagtgt ccgattcttc 15300
tattagactg tgagactctt ggtcatatag tttggatctg tgtccctacc caaatctcat 15360
gttgaaatgt aacccccaat gctggaggtt gcctggatca tggaggttgg aagtgattgg 15420
atcatggagg cagattctca tgaattaacg ccatcccctc ttggtgctct tgtcacgata 15480
gtgagttctc atgaggtctg aatgtttaaa agtgtgtggc atcgccccac cacttctccc 15540
cacgtgtgag acgcacttgc tcctgctttg ccttcagcca tgagtaaaac ctccgtgagg 15600
cctcgccaga agcagaagtc actaggcttc ctgtacagcc tgtggaacca ttagccaatt 15660
aaacctcttt cttataaatt aaccagtctt agatacttct ttataggagt gcaagaaggg 15720
actaatatac ttggggagaa ggcagtgcat gatttatctc tgaatgaggg gccagaaaca 15780
caggaggctc acaatcaatg tctgctaaat gaatgacgtt ataaacttat ctctgatttt 15840
aagaaatgaa ggacatcatt ccttcaccta gtttaaatgt ccatgtttcc agaaagtcta 15900
tagaagcaat tccaatggtg gtagttgggc aacagcagct aatagggtac gtctcttctc 15960
caattatggg caagaactga ctacagcagt ggacagggct cctcctggcc tatctcagct 16020
ctgaagggat gtcctaggaa gactcagacc actatggctg gcgttaacct ttttggcaat 16080
caaagtaagg aatgggagag tttaaggcca gaacttggtt gttctcctaa gttcccccca 16140
aatctataag ccctccaaag ggatgcactg cagccaggga gggcaccccc agatgtaccc 16200
tcgggacccg ccatgagaat ccacgcataa gagggaaggg tcagaccaac tgcctcaaac 16260
tgagaatttc caacctgtga gagagaagtg gagggaaggg atgaaaagtg ggagaaacga 16320

tggagaacaa gaatgactag agacagtagt aatgttcttt atgtaaacag gtgataatag 16380
caatgacaaa attaagaaaa taaaaacact gccatgaaga tttatagact agctaacaca 16440
gagctaaatc ggcaataact tgggctgata aaaggcaacg gggcatcagc cacgtaaaca 16500
gctcgtcact tcaatgcttc cgtgtatatt atgatgacac tggtttgttg ataccacacc 16560
gcccccatga ccccaggcac cagcagtggg gcctgactca ctgtggggag gttgtgcagg 16620
gttgacactg aagagtagct ttttcggaaa gacaagttat ccttgactgg atgcagatgg 16680
ctcggtgaaa aatgaaatct gccagcagac actgccttta tcagctgtag aagttcagca 16740
gggacgagac ttccccatcc ctggcccgcc tgaacaagcc tctggtttca gcaccatatc 16800
acaggctgtc ctaatgaatt cttctgcggg gttggagcct tcacatatct gcttctagga 16860
gtgcatcctg gcgtctacag catggtacgg aactcgcctg gtgtttgcat tccgcctcat 16920
ggagttgtca aggaatgctt gcagcaggaa agggagtgcc agccttacca gggcattggt 16980
atgccctgcc tcaactgggc catcttgcca gtatcctggg ccaccctcat tgacttccag 17040
gtgaggggtg cccaggaaaa accaagaggc tccccaggac atgtcaagcc agacaaccat 17100
tagtgtctac agtgtggaag acagtaataa gcaccactgt taattaactt tctcttatga 17160
gctgaccccc atgccaggca ctgtgtctca tgtaacacgc atctgtccca tgaggttact 17220
gttatagcct cgcctcatag atgggcaaac tgagggctac atggacctgt ccagaatcag 17280
ggggctggga agtggcagat ccagggaata aaatggagct tcatatatct acagagcctg 17340
taaccttcaa aggacaaata agaatgaaaa tgggcaaaat aagactaaat atctggagat 17400
ttggcaccct acagtcagat aaatgagcag atgcttcggt ttcactcgtg atgcagttgc 17460
atcattgaga aggccgcagt ctgacagatg aatctaccat gtggatgttt tttaaacaca 17520
ctataaattc atttcggaac acagcacctg aatgtttgaa ttagtgctgc ttgtggttat 17580
ttgtgtcact gttctgctcc tttgaaacca gaacttagga ggcaaggtgt ctccaaggcc 17640
ccaaatcgcc ctgtgaactg agcccgattc cttgatactg tccctcctgc cgacacactc 17700
aggtgagaaa ggtgtacttg tcccaagtgc tttccatgcc ttcttcttct cctcttatct 17760
gggaagttga aactggcagg aggcattctg atgggttcag agaagacctt tatacaaagg 17820
atgcacagaa caattgaggc atgggcctga cctccaatga gggtgggtcc cagccagtcc 17880
cctaacagca ccttcctctc tggggtgaga cagatgggca agtgtccctt ccacttcctg 17940
acttgtgcag gtcaaaggag aacagagaca actgtgtgac gagggagttt ggcagtgact 18000
gaccccggct cctgcctgct ccgcccagcg gtgcccagct ctcctctctg cagtgaggca 18060
gggccaaggc tccctggcag gagtggcgca gggccagtgc aggccggggc ccagcatcag 18120
gctctgcctc agtcctttct gaatggctgc acctgctcca cgcacagcca gaggcctgac 18180
tgccctgagc agtgcctgcc agcaagaccg agggtaggcg ggctaagcac ctgtggcctc 18240
tcctttcatc tttaggacaa cctgggaggt gaagagtggt gctgtctgag ttgaagctcc 18300
aaggcacaga aggcgttgcc gggctcacgg cagtgagtgg cagagggtgt gctggtagcc 18360
actgcacctc agcactggaa tgttcctgtc tctttggggg tgggggccat gccaccccat 18420

tgcctcttcc cacaatcctt accctttgct ctggtccctt tctaggctgt ccttggtgag 18480
cagaggtgag cagagactgg ccaggggctg gagcaatagg cagaactgtt gtggcatctt 18540
gccactagcg gttgaatgct actaacactg gtggggttgt ggggaggggt gtccttcaaa 18600
gttctcctca gtggtgttcc ctggggcctg ggaacccttg cctagaaaca gtggtggttt 18660
gtctccatga ggcagcatca ggaacatctc tctcatcagg cagcatcggg aacattcact 18720
ggatgggcac tttgtgttgc ccaccgagtg aattcaaagg cagctctctc aggagggaga 18780
ggtaaatgct gtctccccca gcctggttat gggtgaaggc agaagctgtc ccacccagct 18840
cctctcgcct tcttcctcca caacgagctc tccatgagcc agagcaggga gcactccttc 18900
ccaggacgct gctaggggag aggtttgcca gggcagggct ggaatgcggg caaagataga 18960
ggccaccgac agggatcgtt atacttttag aagtcagaaa aattgggaca ggcggtgtct 19020
ctaggacagg ctccctggcc tttttcattt ggatggtaag aactcatctt ttaaaagtgg 19080
aggctgcctg ctgagaagtg ggacaatccc atccatgggg tcacgtccgc aagagtcccc 19140
cgcacccctc catccctgga aaccaacagc ttcctcctca gcccgctccc tctgacgtag 19200
aactccttgg gaaagcagga tactgctttt aaagagtaat cattgacctt gaactcttaa 19260
gatgtgtttc tcaattttcc ccttttatca ctatccggct aacattatgc tgtgtcgttt 19320
cttgagttcc ggtatttaat acaaaatgtt gaatgaaaac atattttgta aacaggctct 19380
acaactgggt atgggagaag ggaacattgt gtcaatcctg cccgtccttt ctctaggcac 19440
ttgaaaaaca tccctggtat cccagcaagc aggcaagata tcactgttgc attctctcaa 19500
cattcctgct cctctggtgc ataaggccaa cgtgcctggg acaaatgtgg tacagtgtag 19560
gagtgaagaa tatgggctgt gactcagtca gattaaaatc tcaactctgc cacatgcatg 19620
aactgcatga ccctgtggga ggtacttttg gagtcttggt ttcttcatct gtaaaatggg 19680
aaccactatc aaaaggaggt gagtggagag actgcatgta aatggagaat gcatgtaaaa 19740
tggcactgag atgcacacaa aggatcaata catggtgtgc attgatccga tttgccgttc 19800
atcacttgag catctcctaa ttgtgaaagt cctggggtgt gcgttcacag gctggtgcag 19860
gttcatgcta ggggcaggcc ctgtgagaca gaagccccct cttcctgcag acctccctga 19920
catccgcact ccattcctca tccgtctcat ctttcttccc agactggtct tcctcactcc 19980
acgcctcctt ctttctcctg gagcccaact gcctcctcca tggccctcca agtccccact 20040
gcatcaggca atccttcatg gccaagtgtc agcatcacca atgcttctgg gacgacagga 20100
gaggggaaag ccactggtct caaccaacct ctcccctctg catttatctc agcccatccc 20160
ccagaccatc cttcaatcct cccctactcc ataaaaatat tgcaccaact ccgcagctca 20220
catttgctca tttggtctgc ttgacttgct taccgctgcg gtctccctgt ctgaggcctc 20280
tactcgaatt gggataattt tgtctgtgct taaaggcact gaccttccag atccccggac 20340
ttcaggaatt tgtcctcatc ccctcatcag tctctgaagc acaaccttat tcagggatct 20400
ggtcttgatc gtcctaactg tacagggctg gccagtgggt ttccagacct ctaaattact 20460

atacccttcc tatgctgtct ggctctctct ttccttctca aggccagcct gagagctaac 20520
agaatcagga ttttgcttga aacatggagg aaagcccaga gccctccatg gaatcagcta 20580
ctttctcagc ataacatcaa ccgacaatag aaatcagacc aggtgtgatc atcatcatga 20640
acatttatat ttatttatca gttttgcaag atcctatcaa ataaataatt tcacatattt 20700
catccactcg ataattaaaa tagtcagtag aagaaaagac ctgaatgaag agcccagacc 20760
tttatatttt catagggatt tgtttccctt tttgtggttc tcatgcactt acctatgaga 20820
aattcctaca agccactctt cctgtttcaa gactttgaag aacaatgcct cctgcgagtc 20880
agttaattca gaatggtggg ttcaagacag cccagacagc aattcttagg ccagccaaga 20940
tgcaccccat cacccctctc atctggtaac agtccatcta cggcaagcaa cttccctctt 21000
ctatgccagg gtctctcaac caggggacta ttgacatttt gggccagagc tttctttggg 21060
aagggggagg aaagtgctgt cctgtgcact gtaggttgtt tagcagcatc tctggcctgt 21120
atcccgcaga taccaggagc accccacccc tgccctggtc atgatgatcc aaaatgtctc 21180
cagaattgcc aaatatcccc tgggggagca aaatcaacca cccgaccacc acccacccac 21240
acacacaccc aaacttcttc ctggcacatc cgttagtcca agatcagtgt tgcaaacacc 21300
cttcagggag gacagggctc caaggaccat cacctttgct ctttgttacc caatagagcc 21360
aagaaagtag ttcctagcag cagcaagaca tgggaaaacg ctgcctgcca gtgaggcttg 21420
gggctcgcaa ggaatacatc ctcaccaagg ccataagact tcaatttgtg gatgctggaa 21480
aacagggcat cagggaggca ggggtccact gctgagatgt gtgactgagt gaggtgcaag 21540
gactggagag gcaggtatgg accctcacca cacttgatct tacctgtgga agtctcccca 21600
acttgtggaa gtcaccaaca taggatctgc caactgtgtt tgaaaacaac cccctcatgg 21660
attgattccc ctgggctagt gcacacagtt cctctgactg cattcagcat gcgtttcttg 21720
acccacagtg gaccttcaca gcagggccct gggccatctt cgctggctcc tctcaaggac 21780
tagaagggcc cagcaccatc ccaggatagt caagtgcaaa tctcatcagt cacccttcaa 21840
gtatttaaca agcccaccct gtagatcctc gctacacaca gtgtcatcca caggccggca 21900
cagggggaat cccctgggaa cttgtcagaa aagaggactc tcaacgccct gccccagaag 21960
ttctgaatct aagcctgaac tttagcaaat tccccagctg ctgtccatgg acatgaaagt 22020
tgagaagccc taatggaagt aatgcaggaa agggagaaga ggatctcctt ccctctagaa 22080
gctcacactc cagcagagaa gatgggatat ataaaaatga aggtcagtac ggtggctcat 22140
gcctgtaatc ccagcacttt gggaggccaa ggtgggcgga tcacggggtc aggagttcaa 22200
gaccagcctg accaacatgg tgaaatcccc atctctacta aaaatacaaa aattagccag 22260
gcatggtggt gcacgccagt aatcccagct acttgggaag ctgaggcagg aaaatcgctt 22320
gaacccagga gttggaggtt gcagtgatcc aagatcgcgc cactgcactc cagcctgggc 22380
aacagagcga gactccgtct caaaaaaaaa aatacacaca cacgcacaca cacaaattag 22440
ctgggcatga tgccatgagc ctgtaatccc aactactcgg gaggctgagg caggagaatc 22500
gcttgaaccc agcaggcaga ggttgcagtg agccgaggtc acaccactat actccagcca 22560

aaaagaaaaa aaggatatat aaaaatgagg caagggagca gagtgcaagt ctacaccacc 22620
aagcatgttt tgggaggacg acacctggat gctgaagacc aagactaaga gctgccttag 22680
ccacttactt ctactgagcc tcaatttcct catctgtaaa gtgggaataa cacttgccct 22740
gtctacctta caggattgtc ctaaagatca aaaaggatgg gaaagcacta ttctgatcta 22800
aagtactatt taatgttaaa ggtactctaa atgctacaga ggttcagaga agagagggct 22860
ccgggtgact ggaagagtct gggaagatcc agcagaggga gttggggagc tgggagtact 22920
gagtcatatg ctaaaccagg tgctgcagta aataaagcaa gatggaaaag tgcacatgct 22980
gaccactgca cccaacaccg cctcaagcct gggccacagg gagctcccac tgacctactt 23040
tcacccacta actcctcaca gtctatggaa gagtaacaca gtgggtctca gaaacacata 23100
cagtgttcac aagtgcttta cagaagacac agagcaagca gagatggtct ctgtggcaag 23160
gctggcaaga agctcaattg ctgcaaaagt tattaactct gggttggggt agagggcaat 23220
ggacttttta ccatttttca gagctgacct gtgaggatgg ctagatactg caagaggatg 23280
cctggaggag ggagaggtaa aagaaatgag ccagtaattt cttgggtaat agttttccta 23340
gataatgttt atgagcattt gctatgtact tgatgttttc atacttaaaa aaaattaaac 23400
ctcactatga tcctatcagg cagggacttt tgtccccact tcacagataa agaaactgga 23460
gatttagaat ttgaagccag gttggtctgc tttccatagc ccatgatctt aactgcaagc 23520
taaacttaca gggtcgtctg agaatctctg ttcttccctg tgcagttgaa acaatcaatg 23580
tggctgtact tttgttgtaa gacctggctc tttccgactc gctgacgttt gccatgcttg 23640
tcctgagctt ctccagagaa atgctgtctt ctggcaggaa aagataaaaa cggctgatat 23700
aaatgagtca ccacactcgc tataattaac agtgatgcaa gagccagatg caaaataaga 23760
ggggcctctg gacctgactt ttaggctgcc caaactttga gctgcacttc aatgtaaaac 23820
agtctccata gctaggcttc catctgtttg agacgcattt taaatgaaga aataaaaccc 23880
ggaatgcatg caagccccaa gataatggag ctgagagggg ctgcaccaaa ttaatctcct 23940
tatttacaat tcaaaaacca tacaccagaa cagaaagaga gtttatagtt tatagataat 24000
atagatcttt gctttcacac tcagttgccc tggaaaggct gagcagtttt caatctgctg 24060
agctgattta tagcaagtac agctgtaaat tcaattgtac taggtgttca gcctatgccc 24120
tcccagtgat gtatctgaac ttttcaaaag gactagttgt cattaacatc tctctatttt 24180
cttttgctga tctgggatga tgggatgcaa tgacaatctg cttaaaataa accttaaaac 24240
cttaaaacat aatcaccaca tcatccacaa accccattca accacagcat gagcacaatc 24300
actctgtaac acaaggtgaa gatattctct gttgcttcta ggacatataa gtccctcctg 24360
aagtttgaca gagagaaata aattgtggtc aggacctgga taggctcact agtagccgtc 24420
gatgtcggtg gcgtcttctc ttacctgttt aaaagaggct cagtggcctt gagatttgaa 24480
cacaatacag ctgatggaat cttgccactt tccttttgaa tagactttaa ttgagagcaa 24540
agtgaaaaat gcaggttaca attttaagtg ttatcatcaa tatcttgtaa gatcaacacc 24600

cagtgatgca tctagtactt atacagtcct aattttagtt gtcaaagagc aaatcattat 24660
cagacatata tttacacagt aatttcaagc aacggtaaat aataaattcc ctcccttaaa 24720
aaacaaaaca caacacaaca cacacacaca cacacacaca cacacacaca cgaccatctt 24780
taaacaatgg atttcctcac ccagtaagtc tcattttttt cacatatact tgtagaccca 24840
aaggtcctta agttacttaa aaacagtctt atcagtggag atcctggacc atcccctatt 24900
ttcttttaaa gtcactttca tttttacaac ttgaagccat ataccagcat ggtttttata 24960
cactaaggac actcccagtg atgaaaatga attcccctcc atttgtgcaa ggaggtatgt 25020
acatctataa agacacatat gattaccact caaaagagtc aaaaacatac acaaaaaaat 25080
ctcagatctt catagtttct taagagcagt gtgccagtgt tcacattgaa ttaactgaat 25140
ggtaaaattc ttttagattt ctatacagag gtactggtta gttctgattt tttcttcagc 25200
tgcttgaaat gcatgagctt taaaagttgc ctggcagaac tacatcctgc tttccaggtt 25260
agcagcctgg ctgacctcgg gaatgttaga caagattcac ccccgatgcc ttgctctctg 25320
atttatttct tgctgttgct gttagtaaat ggaaaccatg acaaagttta cattagcaga 25380
cacatacaca tgccctggcc tataaatatt ccatgataga aaatggtaca ataattccat 25440
ccccagcgaa aacgagcata aaaaaagaag ggaagggtca gggggacatg atcagctatg 25500
gctcttctta cagtagaaaa tccacaccaa atggcaatgg gcttttaact aaccagacat 25560
tgttagctgc ttctccaggg cctgtgagtc aactaaaagt aggcagggca gagaccaact 25620
cttccaggca cacctggtag tttccagaag caccggtatc ttgtcttgga agttggatga 25680
agaagatcaa ctgaagtttc ctttttagtg aggaggagtg gagagtcaga cttgaaagca 25740
gttcacacac tgtgctgcct gggacactga acggtccccg agggagaccc ctcaaagcgc 25800
cagctggagt ttggtcatgt ttctctgatg catgttgtga tggcggacta attcatctga 25860
ccgggcaaac tttttctgac aacttggcca ccgacagctg aagggctttt cacctgttga 25920
cacaattgcc agtcagagac acttgcaaca aagagacagg cacaagttca actatcaagg 25980
cccgagtgaa gtcatcacaa ggcacccact ctgaatttcc catcattctg tccctgagct 26040
aaccaactga gctcatttcc caggcagcct tgaaacagca gcacctcttg gggctttaag 26100
gatcgaaata ttgtaaaaag ttatattctt aggtcagaaa acttggaaag tgggagataa 26160
agaatgagtc aagttggggg aaatgtctga gcaattcata attaagggga aaaacgtttg 26220
tttcagtctc tgctgataga atgttttgtg aactgtgtaa atttctcccc cttgttgagt 26280
aagttgggaa ctgagatgaa ctgggctgca gaagcctctg ggtcatctac cctgggtatt 26340
actgagtgat cactgtccag ggatctcagc tgtgactgaa gcttttaaca cttctatctg 26400
tgtagatacc ctggatgggc tggatacttt aaaataaatc actgatacca gtttacatgt 26460
tatgtgttgt tttttttttt ctcttaaagt gttactgaga gcataatcct gttatatgga 26520
agattctagt tggggcgtgg ttcttagatt tttaataagc ttgccaaaag attgaggctt 26580
ctccacccaa agagccctca gacattcttt ccaagcctca ttctcattag ggagaataac 26640
taccatttat ttgatgctta acatatgcca agccttgtgc tgggcttcac acacgcacac 26700

acacaacctc atgtactcct tacaactgcc cgtaggggaa gcgttattat ccccactcta 26760
cagatgaggt aggacatcta ttgagggcag atccaggcag aactcaaatc tccaggtctg 26820
tcgaattcca gagcccgtgc tcttccctgt atcacacctc ccaggcctag ccatgatgga 26880
aaagcagctg cagctccatc ttaaggaaga atgcacagaa gctaaaccct gtgccacctg 26940
acggatcttc tcagatactg ctggtcccac ttggccttct ctgagaacta ctggtttgga 27000
caagcactga gaaatgtccc aggagggctg gtgtcccagg gtcagactgg aggccaatca 27060
aatgtggtat taaacagtct gctggcaaaa caggtcatgt cacacttact tggggagtgt 27120
gacagccgtg tcaaagggac tataacactt tgccaccgaa ttatgagctc aggactaagt 27180
taaagaagca tcaaaggcac ctgaggaagg gaagcaagat cccatcctgc caaccatggg 27240
ccacaggtgc cttggctctg tttttaaatg cagccaattt gggcgaagaa tctggggctg 27300
ctcaggggtt tgtgatgtca tgttttcctt cactcgctgt tcctctgctc ttcctggcct 27360
ggctctcact ctccaacccc acctccagca gtgctgttaa gggctggctc ctaatggact 27420
aatcccaacc ctgtctcttg tcatcttcat tcctctcagt ctctcagtgg tgttcagctt 27480
tgctgacttt gattctctac ttgactctcc atgctccctg gactgcagag taaatatctg 27540
ccccttctcc aataaagaag caagtgggac aaggtgggct ggaagcagac agaactggat 27600
ttgctcctta ctagctttgt gactttagat aagccattga attattattg cctcagtctc 27660
ctcatctgta agatgggaat aaaacatgcc tacctcattt ttttttgttt gttttgtttt 27720
gttttgtttt gagacagggt cttgctctgt tgcttgggtt ggagtacagt ggtgggatca 27780
cagctcactg cagcctcagc ctcccaagca aggtctagtg agcgtcctac ctcagcctcc 27840
cccaaatagc tgggactata ggtccacacc accatgccca gctagttttt gtattttttg 27900
tagagacagg gttttgccat gttgcccagg ctagtcttga actcctgggc tcaagtgatc 27960
cacccacctc atcccaaata gctgggatta caggcatgag ccaccacagc cggtcctacc 28020
ttactctact tacaagcatg ttgtgaagat agacttagat aagctaaata ttaacgctct 28080
ttccaaaggt agaaaatatg caacaaaggt ggtgctattt ttattatgaa tcacaccagc 28140
cttgcagaaa gcaaaaacct tcagcagcca gtaaatgaat gccctattct cctctctgta 28200
ctagtatgaa ggaaataata tccacacact gatactggtc cagcagaaac caagaccgct 28260
cctggtgcat taacttttaa cagagcaagg actcagttct ctgaaaatag tgccattaaa 28320
catgtgctct cagaagaata aatatttggc ttgctagaat ttctgctgct tttctgtaaa 28380
gttgattatc gtatcaaaga gagtaacaaa atgtgtgatg caacaaactg gaaacagtag 28440
ggacctggct tatctcttca ttttcattct gcacactcca gccattcttt tcccatcttt 28500
ctccttctct gtatttccac cctccccttc tttaaaaaat gttattattt attatttaaa 28560
gatagccacg cactattcct tctctcaact gagtctaaac cttagaactt ttacactagt 28620
cttttccaat ccctctcatc acaatttcat tccacaatag tttaaaaaaa taatgaaaaa 28680
taaatgtgaa gaaaagttta cgcacttgtt ttacctgtat gagtcctggt gtgggtcttc 28740

aggtggtcgg accgggagaa ctttcgctga caagttttac actggaatgg tttcacacct 28800
aaatggacag agaaggtcta gcctcggccc taacaatgtg ggcacagtga ggcccacaag 28860
gctgacttcc ggcagctgga ggagcccagc atttcctgcc atgcctgcaa tgtctgcatc 28920
tgcctcaccc ttagatttcc ccagtccccc aggcccaaca agaatcactg attctgcaca 28980
gtaatcttca aggacctgct tctgtcttgg gaacaaccat tccttaacca cagaggtcca 29040
gcttctcacc agctggaaga tctcagctgt gtcagagaca ggaaaagtga atcacacgct 29100
acaaattgga ttccgctctc catcaccctc attctttgct gatgctcagt gtatcatcag 29160
cccactgcta gtgggatctc actgttctgt ggtttgcagg ggaaatgtgg ggtgtttcct 29220
tttctttctt tttctttatt taagaggagg aacatctcca gagattatgg ggggaaaata 29280
ctggaaaaac taaacacatg gctgactctc tcattcatat tcaacaacaa agagaatcat 29340
gaaatcaacc ctagcccaag ggaacacagc tgccagcaat gagaagtgaa cctacaaacc 29400
tgtatgtctc ctttggtgtc ttttgagctg gtctgaacga gaaaaccttc gttcacagtc 29460
cttgaagtca cactggtatg gtttctcacc ttggggaaga cacatattct atttgaaaat 29520
gatactggaa aaggggatct cattaaaggc aacctctcct actaggactg aacagatccc 29580
aaaatgccta aggcacttcg ctggagcttg ttagggtagg atgatcccca acttgcaagt 29640
tacaaatgaa tgggagctca ggggccagag gattctgccc caaggctcac atctagctct 29700
gccattaaaa aaaaaaaaaa agccctgggt gaggtgtggt ttctttattg taaaatgggt 29760
ccaataatcc aacaacttta aaggatttat gaccttcaca taaagggctc agcccagagc 29820
ctggcataga aggcgtgctc aaaaactatt agcaaataaa aaacaaaatc tttaactccc 29880
aggcagaatc tgaggcagaa ttctgggatg catgtgcctt gaggtcaacc tcatgaaata 29940
agagttttat gttttagggc ccaaagatct ggtgttcttt gctactgact ccttgttctt 30000
gcatcaagca aatatgactc catggttgtg agaccctcaa aacacaccaa aagcactgag 30060
aaagtttttg gtgtgcttta tccaagagaa gtcagtagtg ggattaccca acctcctgtc 30120
tcagaatgga gaagcctcac ccctgtcctc actagagaaa gttcagccaa atactgggac 30180
ctgagcccca agagaagtga gcaaccccag gccttcccac cttcttagga gaatcctgag 30240
tcacaggcat ctgggatgga aacaagccat tctagaatca gtcaggtctt tctgttgcaa 30300
aggcagcccc ctgttacctt tccaatctca aagcctgggc tatttccctc atgaatctct 30360
attccccact atgccagagc tcacatcctg aagtcctcct tgcactctgc tcacactaaa 30420
tgtcccccat ccctatctcc acatcgaaaa gtctcctggg tgtctccttc ttctgcaatg 30480
cctgcttgga aactgcatgg gccaatttcc cttccttccc tcattacgac atttattcca 30540
ggctgtctaa tgtggtgagc tgtgtctggg tctacaattc tacaattctt caatgggttg 30600
gaaaatccta agggctggag cctttttatt tttatttttt ctcagagaca gagtctctct 30660
ctgttgccca gactaaagcg cagtggtgtg agcctagctc actgcagcct cgaaaccgtg 30720
ggcacaagca atcctcctac ctcagcctcc tgagtagctg ggactacagg catacaccag 30780
cacacttggc taaattttta aaaatatttt ttttagagac agggtctcac tatattgccc 30840

aggctggtct tgaacccctt aagcaatcct cccgcctacg cctcccaaca tactgggatt 30900
acaggtgtga gccactgcac ctggcccctt tctttgtatt tgtatccagg ttggtgcctt 30960
atgtttcacc tctgtgagct ggagaagatg ttacccctat tgagtctgat ttccttcccc 31020
tgcctttgag cttctgctca tctatcactt tctctgccca aactaccagc ctccactggc 31080
taacccaagc tgttcttcct ctgagaaact ttctcggatg accttagttc aaactgatac 31140
cctccaaacc cctcggtacc tcctgtctct gatttagtac atgatcatgt actctctcag 31200
tcacttttca tttctttata tcctagctcc caaacaaacc aggaactctt tgagaaggag 31260
gattataacc tgcagttaaa aagaaggagt ttgagtcctc tttctgctat atcctatact 31320
cttcataaat gttttgtgag tgaggattaa atgaaatata catgtagaga gcctgacacc 31380
caaaaagtac ttaatataac agtctgtact gttgttataa ataaataaga aaatattgct 31440
ctctttcaag ggggactgta ggattgttaa ataccaacct gccctcatct tctgcaagga 31500
agagtttctt aaaggctcca gtgggtctgg ggttttggct gtcttgttca ctgccttccc 31560
ttcacacagt tgtctaaccc tcactgcatt ctaagtgctt ctctctcact aaaagtttct 31620
acaaccatat tgccttcatt tattcccttg catttttcag gcaggacttt actttctcgt 31680
tttccttata accttgacag tcacttttca aatgatccca tttggggaaa tgtgtaaaaa 31740
cataacatgc cactggtccc ctccttttta gcctcaaagt ctgtggccca ctgagtgcaa 31800
actcagattg acaccagaat tgcatcctct ggacacagtg aattagcaaa tttcaatggt 31860
ccaaggaaat ctgaatgcag ctcacttttt tctccagctt cccaagagca aaatcagcag 31920
taataacaca gttgcaacag tgatcctata ataatgctgt tcttcgattg tggaaaaggc 31980
agcagtagct cctctgtagg aaaacctggg ttttaccaca ctcccacact cccacctagt 32040
gcccacaagg cccagagtaa acgttctgtg acatggctgt ccacattcaa ggtggccaac 32100
accaaacaat gccagttctc tgtttcccag aagcagcctc tctgacctgg gggaaaatcc 32160
agtttcattg gcgtatcttc aaagataaaa aatcatcatc tctctctctc cttttttttt 32220
tttttggcag agtcttgctc tgtcgcccag gctggagtac agtggtaaga tctcagctca 32280
ctgcaacctc tgcctcctag gttcaagcaa ttctcctgcc ccagcctccc aagtacaggt 32340
gcgtgccacc acacccggct catttttgta tttttagtag agacggggtt tcaccttgtt 32400
ggccaggctg gtctccaact cctgaccttg ggtgatccac ccacttcggc ctcccaaagt 32460
gctgggatta caggcgtgag cccccatgac tgaccttttt tttttaagtg atcactaaaa 32520
aatcaaacta acaatcaatt ttccccttcc tgtctagttg attattactg acccaagcag 32580
gtaagttttt ctttggacca tctcttctaa aatttcacaa tgatcttgcc catcgacctc 32640
ctaaaaccat ctgatgacta tattcccagg ccagctcctc tctccttaga aatgctaata 32700
gtattctaac accaacacga ttcctacaca ggtctcttta attctgtggc actttctctc 32760
taccactctg ctctgccttt ctggaagaca gtttactgac ctctgtaata aaggatagat 32820
aatcagaatg caaaatggcc ccacagcctc tttacaacac ctggatcaga cctttcaaag 32880

cagtgcttac tttccatcct ggaaataacc tgggtcctta gcagtgtgag agcctggaaa 32940
aggagctctt gaaccatgtt tgcccaagac tggacagcgg gcacacttac cagtgtgctt 33000
cctgctgtgc atctgtaagt gggacagctt aaaatatctc ttattgcagc ctgggtaagc 33060
acacatgaag gggcgtttct cactggtctc agatgccgac cgtacaagag tcggggctac 33120
tccaggcaca cgt 33133